# EUROPÄISCHE PATENTSCHRIFT

(11) **EP 1 902 067 B1**
(45) Veröffentlichungstag und Bekanntmachung des Hinweises auf die Patenterteilung: **26.10.2011**
(21) Anmeldenummer: 06777211.1
(22) Anmeldetag: 03.05.2006
(51) Int. Cl.: C07K 14/34

(54) **ALLELE DES OPCA-GENS AUS CORYNEFORMEN BAKTERIEN**
ALLELES OF THE OPCA GENE FROM CORYNEFORM BACTERIA
ALLELES DU GENE OPCA PROVENANT DE BACTERIES CORYNEFORMES

(30) Priorität: 24.05.2005 DE 102005023829
(43) Veröffentlichungstag der Anmeldung: 26.03.2008
(73) Patentinhaber: Evonik Degussa GmbH, 45128 Essen (DE)
(72) Erfinder: SCHISCHKA, Natalie, 33649 Bielefeld (DE); BATHE, Brigitte, 33154 Salzkotten (DE); THIERBACH, Georg, 33613 Bielefeld (DE)
(86) Internationale Anmeldenummer: PCT/EP2006/062010
(87) Internationale Veröffentlichungsnummer: WO 2006/125714

(56) Entgegenhaltungen:
- EP-A- 1 669 369
- WO-A1-01/04322
- DATABASE UniProt 1. März 2003 (2003-03-01), "Putative oxppcycle protein OpcA" XP002414959 gefunden im EBI Database accession no. Q8FT73
- DATABASE UniProt 5. Juli 2004 (2004-07-05), "Hypothetical protein" XP002414960 gefunden im EBI Database accession no. Q6NH40

### SEQUENCE LISTING

<110> Degussa AG
<120> Allele des opcA-Gens aus coryneformen Bakterien
<130> 050109BT
<160> 20
<170> PatentIn version 3.3
   <210> 1
   <211> 960
   <212> DNA
   <213> Corynebacterium glutamicum
<220>
   <221> CDS
   <222> (1)..(957)
   <223> opcA Wildtyp-Gen
<400> 1
   atg atc ttt gaa ctt ccg gat acc acc acc cag caa att tcc aag acc       48
   Met Ile Phe Glu Leu Pro Asp Thr Thr Thr Gln Gln Ile Ser Lys Thr
   1               5                   10                  15
   cta act cga ctg cgt gaa tcg ggc acc cag gtc acc acc ggc cga gtg       96
   Leu Thr Arg Leu Arg Glu Ser Gly Thr Gln Val Thr Thr Gly Arg Val
               20                  25                  30
   ctc acc ctc atc gtg gtc act gac tcc gaa agc gat gtc gct gca gtt      144
   Leu Thr Leu Ile Val Val Thr Asp Ser Glu Ser Asp Val Ala Ala Val
           35                  40                  45
   acc gag tcc acc aat gaa gcc tcg cgc gag cac cca tct cgc gtg atc      192
   Thr Glu Ser Thr Asn Glu Ala Ser Arg Glu His Pro Ser Arg Val Ile
       50                  55                  60
   att ttg gtg gtt ggc gat aaa act gca gaa aac aaa gtt gac gca gaa      240
   Ile Leu Val Val Gly Asp Lys Thr Ala Glu Asn Lys Val Asp Ala Glu
   65                  70                  75                  80
   gtc cgt atc ggt ggc gac gct ggt gct tcc gag atg atc atc atg cat      288
   Val Arg Ile Gly Gly Asp Ala Gly Ala Ser Glu Met Ile Ile Met His
                   85                  90                  95
   ctc aac gga cct gtc gct gac aag ctc cag tat gtc gtc aca cca ctg      336
   Leu Asn Gly Pro Val Ala Asp Lys Leu Gln Tyr Val Val Thr Pro Leu
               100                 105                 110
   ttg ctt cct gac acc ccc atc gtt gct tgg tgg cca ggt gaa tca cca      384
   Leu Leu Pro Asp Thr Pro Ile Val Ala Trp Trp Pro Gly Glu Ser Pro
           115                 120                 125
   aag aat cct tcc cag gac cca att gga cgc atc gca caa cga cgc atc      432
   Lys Asn Pro Ser Gln Asp Pro Ile Gly Arg Ile Ala Gln Arg Arg Ile
       130                 135                 140
   act gat gct ttg tac gac cgt gat gac gca cta gaa gat cgt gtt gag        480
   Thr Asp Ala Leu Tyr Asp Arg Asp Asp Ala Leu Glu Asp Arg Val Glu
   145                 150                 155                 160
   aac tat cac cca ggt gat acc gac atg acg tgg gcg cgc ctt acc cag        528
   Asn Tyr His Pro Gly Asp Thr Asp Met Thr Trp Ala Arg Leu Thr Gln
                   165                 170                 175
   tgg cgg gga ctt gtt gcc tcc tca ttg gat cac cca cca cac agc gaa        576
   Trp Arg Gly Leu Val Ala Ser Ser Leu Asp His Pro Pro His Ser Glu
               180                 185                 190
   atc act tcc gtg agg ctg acc ggt gca agc ggc agt acc tcg gtg gat        624
   Ile Thr Ser Val Arg Leu Thr Gly Ala Ser Gly Ser Thr Ser Val Asp
           195                 200                 205
   ttg gct gca ggc tgg ttg gcg cgg agg ctg aaa gtg cct gtg atc cgc        672
   Leu Ala Ala Gly Trp Leu Ala Arg Arg Leu Lys Val Pro Val Ile Arg
       210                 215                 220
   gag gtg aca gat gct ccc acc gtg cca acc gat gag ttt ggt act cca        720
   Glu Val Thr Asp Ala Pro Thr Val Pro Thr Asp Glu Phe Gly Thr Pro
   225                 230                 235                 240
   ctg ctg gct atc cag cgc ctg gag atc gtt cgc acc acc ggc tcg atc        768
   Leu Leu Ala Ile Gln Arg Leu Glu Ile Val Arg Thr Thr Gly Ser Ile
                   245                 250                 255
   atc atc acc atc tat gac gct cat acc ctt cag gta gag atg ccg gaa        816
   Ile Ile Thr Ile Tyr Asp Ala His Thr Leu Gln Val Glu Met Pro Glu
               260                 265                 270
   tcc ggc aat gcc cca tcg ctg gtg gct att ggt cgt cga agt gag tcc        864
   Ser Gly Asn Ala Pro Ser Leu Val Ala Ile Gly Arg Arg Ser Glu Ser
           275                 280                 285
   gac tgc ttg tct gag gag ctt cgc cac atg gat cca gat ttg ggc tac        912
   Asp Cys Leu Ser Glu Glu Leu Arg His Met Asp Pro Asp Leu Gly Tyr
       290                 295                 300
   cag cac gca cta tcc ggc ttg tcc agc gtc aag ctg gaa acc gtc taa        960
   Gln His Ala Leu Ser Gly Leu Ser Ser Val Lys Leu Glu Thr Val
   305                 310                 315
<210> 2
   <211> 319
   <212> PRT
   <213> Corynebacterium glutamicum
<400> 2
   Met Ile Phe Glu Leu Pro Asp Thr Thr Thr Gln Gln Ile Ser Lys Thr
   1               5                   10                  15
   Leu Thr Arg Leu Arg Glu Ser Gly Thr Gln Val Thr Thr Gly Arg Val
               20                  25                  30
   Leu Thr Leu Ile Val Val Thr Asp Ser Glu Ser Asp Val Ala Ala Val
           35                  40                  45
   Thr Glu Ser Thr Asn Glu Ala Ser Arg Glu His Pro Ser Arg Val Ile
       50                  55                  60
   Ile Leu Val Val Gly Asp Lys Thr Ala Glu Asn Lys Val Asp Ala Glu
   65                  70                  75                  80
   Val Arg Ile Gly Gly Asp Ala Gly Ala Ser Glu Met Ile Ile Met His
                   85                  90                  95
   Leu Asn Gly Pro Val Ala Asp Lys Leu Gln Tyr Val Val Thr Pro Leu
               100                 105                 110
   Leu Leu Pro Asp Thr Pro Ile Val Ala Trp Trp Pro Gly Glu Ser Pro
           115                 120                 125
   Lys Asn Pro Ser Gln Asp Pro Ile Gly Arg Ile Ala Gln Arg Arg Ile
       130                 135                 140
   Thr Asp Ala Leu Tyr Asp Arg Asp Asp Ala Leu Glu Asp Arg Val Glu
   145                 150                 155                 160
   Asn Tyr His Pro Gly Asp Thr Asp Met Thr Trp Ala Arg Leu Thr Gln
                   165                 170                 175
   Trp Arg Gly Leu Val Ala Ser Ser Leu Asp His Pro Pro His Ser Glu
               180                 185                 190
   Ile Thr Ser Val Arg Leu Thr Gly Ala Ser Gly Ser Thr Ser Val Asp
           195                 200                 205
   Leu Ala Ala Gly Trp Leu Ala Arg Arg Leu Lys Val Pro Val Ile Arg
       210                 215                 220
   Glu Val Thr Asp Ala Pro Thr Val Pro Thr Asp Glu Phe Gly Thr Pro
   225                 230                 235                 240
   Leu Leu Ala Ile Gln Arg Leu Glu Ile Val Arg Thr Thr Gly Ser Ile
                   245                 250                 255
   Ile Ile Thr Ile Tyr Asp Ala His Thr Leu Gln Val Glu Met Pro Glu
               260                 265                 270
   Ser Gly Asn Ala Pro Ser Leu Val Ala Ile Gly Arg Arg Ser Glu Ser
           275                 280                 285
   Asp Cys Leu Ser Glu Glu Leu Arg His Met Asp Pro Asp Leu Gly Tyr
       290                 295                 300
   Gln His Ala Leu Ser Gly Leu Ser Ser Val Lys Leu Glu Thr Val
   305                 310                 315
<210> 3
   <211> 1600
   <212> DNA
   <213> Corynebacterium glutamicum
<220>
   <221> CDS
   <222> (335) .. (1291)
   <223> opcA Wildtyp-Gen
<400> 3
   gtgtgctcat ccgcttcggt tccaaggttc caggttctgc catggaagtc cgtgacgtca      60
   acatggactt ctcctactca gaatccttca ctgaagaatc acctgaagca tacgagcgcc     120
   tcattttgga tgcgctgtta gatgaatcca gcctcttccc taccaacgag gaagtggaac     180
   tgagctggaa gattctggat ccaattcttg aagcatggga tgccgatgga gaaccagagg     240
   attacccagc gggtacgtgg ggtccaaaga gcgctgatga aatgctttcc cgcaacggtc     300
   acacctggcg caggccataa tttaggggca aaaa atg atc ttt gaa ctt ccg gat     355
                                         Met Ile Phe Glu Leu Pro Asp
                                         1               5
   acc acc acc cag caa att tcc aag acc cta act cga ctg cgt gaa tcg       403
   Thr Thr Thr Gln Gln Ile Ser Lys Thr Leu Thr Arg Leu Arg Glu Ser
           10                  15                  20
   ggc acc cag gtc acc acc ggc cga gtg ctc acc ctc atc gtg gtc act       451
   Gly Thr Gln Val Thr Thr Gly Arg Val Leu Thr Leu Ile Val Val Thr
       25                  30                  35
   gac tcc gaa agc gat gtc gct gca gtt acc gag tcc acc aat gaa gcc       499
   Asp Ser Glu Ser Asp Val Ala Ala Val Thr Glu Ser Thr Asn Glu Ala
   40                  45                  50                  55
   tcg cgc gag cac cca tct cgc gtg atc att ttg gtg gtt ggc gat aaa       547
   Ser Arg Glu His Pro Ser Arg Val Ile Ile Leu Val Val Gly Asp Lys
                   60                  65                  70
   act gca gaa aac aaa gtt gac gca gaa gtc cgt atc ggt ggc gac gct       595
   Thr Ala Glu Asn Lys Val Asp Ala Glu Val Arg Ile Gly Gly Asp Ala
               75                  80                  85
   ggt gct tcc gag atg atc atc atg cat ctc aac gga cct gtc gct gac       643
   Gly Ala Ser Glu Met Ile Ile Met His Leu Asn Gly Pro Val Ala Asp
           90                  95                  100
   aag ctc cag tat gtc gtc aca cca ctg ttg ctt cct gac acc ccc atc       691
   Lys Leu Gln Tyr Val Val Thr Pro Leu Leu Leu Pro Asp Thr Pro Ile
       105                 110                 115
   gtt gct tgg tgg cca ggt gaa tca cca aag aat cct tcc cag gac cca      739
   Val Ala Trp Trp Pro Gly Glu Ser Pro Lys Asn Pro Ser Gln Asp Pro
   120                 125                 130                 135
   att gga cgc atc gca caa cga cgc atc act gat gct ttg tac gac cgt      787
   Ile Gly Arg Ile Ala Gln Arg Arg Ile Thr Asp Ala Leu Tyr Asp Arg
                   140                 145                 150
   gat gac gca cta gaa gat cgt gtt gag aac tat cac cca ggt gat acc      835
   Asp Asp Ala Leu Glu Asp Arg Val Glu Asn Tyr His Pro Gly Asp Thr
               155                 160                 165
   gac atg acg tgg gcg cgc ctt acc cag tgg cgg gga ctt gtt gcc tcc      883
   Asp Met Thr Trp Ala Arg Leu Thr Gln Trp Arg Gly Leu Val Ala Ser
           170                 175                 180
   tca ttg gat cac cca cca cac agc gaa atc act tcc gtg agg ctg acc      931
   Ser Leu Asp His Pro Pro His Ser Glu Ile Thr Ser Val Arg Leu Thr
       185                 190                 195
   ggt gca agc ggc agt acc tcg gtg gat ttg gct gca ggc tgg ttg gcg      979
   Gly Ala Ser Gly Ser Thr Ser Val Asp Leu Ala Ala Gly Trp Leu Ala
   200                 205                 210                 215
   cgg agg ctg aaa gtg cct gtg atc cgc gag gtg aca gat gct ccc acc     1027
   Arg Arg Leu Lys Val Pro Val Ile Arg Glu Val Thr Asp Ala Pro Thr
                   220                 225                 230
   gtg cca acc gat gag ttt ggt act cca ctg ctg gct atc cag cgc ctg     1075
   Val Pro Thr Asp Glu Phe Gly Thr Pro Leu Leu Ala Ile Gln Arg Leu
               235                 240                 245
   gag atc gtt cgc acc acc ggc tcg atc atc atc acc atc tat gac gct     1123
   Glu Ile Val Arg Thr Thr Gly Ser Ile Ile Ile Thr Ile Tyr Asp Ala
           250                 255                 260
   cat acc ctt cag gta gag atg ccg gaa tcc ggc aat gcc cca tcg ctg     1171
   His Thr Leu Gln Val Glu Met Pro Glu Ser Gly Asn Ala Pro Ser Leu
       265                 270                 275
   gtg gct att ggt cgt cga agt gag tcc gac tgc ttg tct gag gag ctt     1219
   Val Ala Ile Gly Arg Arg Ser Glu Ser Asp Cys Leu Ser Glu Glu Leu
   280                 285                 290                 295
   cgc cac atg gat cca gat ttg ggc tac cag cac gca cta tcc ggc ttg     1267
   Arg His Met Asp Pro Asp Leu Gly Tyr Gln His Ala Leu Ser Gly Leu
                   300                 305                 310
   tcc agc gtc aag ctg gaa acc gtc taaggagaaa tacaacacta tggttgatgt    1321
   Ser Ser Val Lys Leu Glu Thr Val
               315
   agtacgcgca cgcgatactg aagatttggt tgcacaggct gcctccaaat tcattgaggt   1381
   tgttgaagca gcaactgcca ataatggcac cgcacaggta gtgctcaccg gtggtggcgc   1441
   cggcatcaag ttgctggaaa agctcagcgt tgatgcggct gaccttgcct gggatcgcat   1501
   tcatgtgttc ttcggcgatg agcgcaatgt ccctgtcagt gattctgagt ccaatgaggg   1561
   ccaggctcgt gaggcactgt tgtccaaggt ttctatccc                          1600
<210> 4
   <211> 319
   <212> PRT
   <213> Corynebacterium glutamicum
<400> 4
   Met Ile Phe Glu Leu Pro Asp Thr Thr Thr Gln Gln Ile Ser Lys Thr
   1               5                   10                  15
   Leu Thr Arg Leu Arg Glu Ser Gly Thr Gln Val Thr Thr Gly Arg Val
               20                  25                  30
   Leu Thr Leu Ile Val Val Thr Asp Ser Glu Ser Asp Val Ala Ala Val
           35                  40                  45
   Thr Glu Ser Thr Asn Glu Ala Ser Arg Glu His Pro Ser Arg Val Ile
       50                  55                  60
   Ile Leu Val Val Gly Asp Lys Thr Ala Glu Asn Lys Val Asp Ala Glu
   65                  70                  75                  80
   Val Arg Ile Gly Gly Asp Ala Gly Ala Ser Glu Met Ile Ile Met His
                   85                  90                  95
   Leu Asn Gly Pro Val Ala Asp Lys Leu Gln Tyr Val Val Thr Pro Leu
               100                 105                 110
   Leu Leu Pro Asp Thr Pro Ile Val Ala Trp Trp Pro Gly Glu Ser Pro
           115                 120                 125
   Lys Asn Pro Ser Gln Asp Pro Ile Gly Arg Ile Ala Gln Arg Arg Ile
       130                 135                 140
   Thr Asp Ala Leu Tyr Asp Arg Asp Asp Ala Leu Glu Asp Arg Val Glu
   145                 150                 155                 160
   Asn Tyr His Pro Gly Asp Thr Asp Met Thr Trp Ala Arg Leu Thr Gln
                   165                 170                 175
   Trp Arg Gly Leu Val Ala Ser Ser Leu Asp His Pro Pro His Ser Glu
               180                 185                 190
   Ile Thr Ser Val Arg Leu Thr Gly Ala Ser Gly Ser Thr Ser Val Asp
           195                 200                 205
   Leu Ala Ala Gly Trp Leu Ala Arg Arg Leu Lys Val Pro Val Ile Arg
       210                 215                 220
   Glu Val Thr Asp Ala Pro Thr Val Pro Thr Asp Glu Phe Gly Thr Pro
   225                 230                 235                 240
   Leu Leu Ala Ile Gln Arg Leu Glu Ile Val Arg Thr Thr Gly Ser Ile
                   245                 250                 255
   Ile Ile Thr Ile Tyr Asp Ala His Thr Leu Gln Val Glu Met Pro Glu
               260                 265                 270
   Ser Gly Asn Ala Pro Ser Leu Val Ala Ile Gly Arg Arg Ser Glu Ser
           275                 280                 285
   Asp Cys Leu Ser Glu Glu Leu Arg His Met Asp Pro Asp Leu Gly Tyr
       290                 295                 300
   Gln His Ala Leu Ser Gly Leu Ser Ser Val Lys Leu Glu Thr Val
   305                 310                 315
<210> 5
   <211> 960
   <212> DNA
   <213> Corynebacterium glutamicum
<220>
   <221> CDS
   <222> (1)..(957)
   <223> opcA-Allel
<220>
   <221> mutation
   <222> (319) .. (319)
   <223> T → C Transition
<220>
   <221> mutation
   <222> (657)..(657)
   <223> A → C Transversion
<220>
   <221> mutation
   <222> (697)..(697)
   <223> C → T Transition
<220>
   <221> mutation
   <222> (781)..(781)
   <223> T → C Transition
<400> 5
   atg atc ttt gaa ctt ccg gat acc acc acc cag caa att tcc aag acc      48
   Met Ile Phe Glu Leu Pro Asp Thr Thr Thr Gln Gln Ile Ser Lys Thr
   1               5                   10                  15
   cta act cga ctg cgt gaa tcg ggc acc cag gtc acc acc ggc cga gtg        96
   Leu Thr Arg Leu Arg Glu Ser Gly Thr Gln Val Thr Thr Gly Arg Val
               20                  25                  30
   ctc acc ctc atc gtg gtc act gac tcc gaa agc gat gtc gct gca gtt       144
   Leu Thr Leu Ile Val Val Thr Asp Ser Glu Ser Asp Val Ala Ala Val
           35                  40                  45
   acc gag tcc acc aat gaa gcc tcg cgc gag cac cca tct cgc gtg atc       192
   Thr Glu Ser Thr Asn Glu Ala Ser Arg Glu His Pro Ser Arg Val Ile
       50                  55                  60
   att ttg gtg gtt ggc gat aaa act gca gaa aac aaa gtt gac gca gaa       240
   Ile Leu Val Val Gly Asp Lys Thr Ala Glu Asn Lys Val Asp Ala Glu
   65                  70                  75                  80
   gtc cgt atc ggt ggc gac gct ggt gct tcc gag atg atc atc atg cat       288
   Val Arg Ile Gly Gly Asp Ala Gly Ala Ser Glu Met Ile Ile Met His
                   85                  90                  95
   ctc aac gga cct gtc gct gac aag ctc cag cat gtc gtc aca cca ctg       336
   Leu Asn Gly Pro Val Ala Asp Lys Leu Gln His Val Val Thr Pro Leu
               100                 105                 110
   ttg ctt cct gac acc ccc atc gtt gct tgg tgg cca ggt gaa tca cca       384
   Leu Leu Pro Asp Thr Pro Ile Val Ala Trp Trp Pro Gly Glu Ser Pro
           115                 120                 125
   aag aat cct tcc cag gac cca att gga cgc atc gca caa cga cgc atc       432
   Lys Asn Pro Ser Gln Asp Pro Ile Gly Arg Ile Ala Gln Arg Arg Ile
       130                 135                 140
   act gat gct ttg tac gac cgt gat gac gca cta gaa gat cgt gtt gag       480
   Thr Asp Ala Leu Tyr Asp Arg Asp Asp Ala Leu Glu Asp Arg Val Glu
   145                 150                 155                 160
   aac tat cac cca ggt gat acc gac atg acg tgg gcg cgc ctt acc cag       528
   Asn Tyr His Pro Gly Asp Thr Asp Met Thr Trp Ala Arg Leu Thr Gln
                   165                 170                 175
   tgg cgg gga ctt gtt gcc tcc tca ttg gat cac cca cca cac agc gaa       576
   Trp Arg Gly Leu Val Ala Ser Ser Leu Asp His Pro Pro His Ser Glu
               180                 185                 190
   atc act tcc gtg agg ctg acc ggt gca agc ggc agt acc tcg gtg gat       624
   Ile Thr Ser Val Arg Leu Thr Gly Ala Ser Gly Ser Thr Ser Val Asp
           195                 200                 205
   ttg gct gca ggc tgg ttg gcg cgg agg ctg aac gtg cct gtg atc cgc       672
   Leu Ala Ala Gly Trp Leu Ala Arg Arg Leu Asn Val Pro Val Ile Arg
       210                 215                 220
   gag gtg aca gat gct ccc acc gtg tca acc gat gag ttt ggt act cca       720
   Glu Val Thr Asp Ala Pro Thr Val Ser Thr Asp Glu Phe Gly Thr Pro
   225                 230                 235                 240
   ctg ctg gct atc cag cgc ctg gag atc gtt cgc acc acc ggc tcg atc       768
   Leu Leu Ala Ile Gln Arg Leu Glu Ile Val Arg Thr Thr Gly Ser Ile
                   245                 250                 255
   atc atc acc atc cat gac gct cat acc ctt cag gta gag atg ccg gaa      816
   Ile Ile Thr Ile His Asp Ala His Thr Leu Gln Val Glu Met Pro Glu
               260                 265                 270
   tcc ggc aat gcc cca tcg ctg gtg gct att ggt cgt cga agt gag tcc      864
   Ser Gly Asn Ala Pro Ser Leu Val Ala Ile Gly Arg Arg Ser Glu Ser
           275                 280                 285
   gac tgc ttg tct gag gag ctt cgc cac atg gat cca gat ttg ggc tac      912
   Asp Cys Leu Ser Glu Glu Leu Arg His Met Asp Pro Asp Leu Gly Tyr
       290                 295                 300
   cag cac gca cta tcc ggc ttg tcc agc gtc aag ctg gaa acc gtc taa      960
   Gln His Ala Leu Ser Gly Leu Ser Ser Val Lys Leu Glu Thr Val
   305                 310                 315
<210> 6
   <211> 319
   <212> PRT
   <213> Corynebacterium glutamicum
<400> 6
   Met Ile Phe Glu Leu Pro Asp Thr Thr Thr Gln Gln Ile Ser Lys Thr
   1               5                   10                  15
   Leu Thr Arg Leu Arg Glu Ser Gly Thr Gln Val Thr Thr Gly Arg Val
               20                  25                  30
   Leu Thr Leu Ile Val Val Thr Asp Ser Glu Ser Asp Val Ala Ala Val
           35                  40                  45
   Thr Glu Ser Thr Asn Glu Ala Ser Arg Glu His Pro Ser Arg Val Ile
       50                  55                  60
   Ile Leu Val Val Gly Asp Lys Thr Ala Glu Asn Lys Val Asp Ala Glu
   65                  70                  75                  80
   Val Arg Ile Gly Gly Asp Ala Gly Ala Ser Glu Met Ile Ile Met His
                   85                  90                  95
   Leu Asn Gly Pro Val Ala Asp Lys Leu Gln His Val Val Thr Pro Leu
               100                 105                 110
   Leu Leu Pro Asp Thr Pro Ile Val Ala Trp Trp Pro Gly Glu Ser Pro
           115                 120                 125
   Lys Asn Pro Ser Gln Asp Pro Ile Gly Arg Ile Ala Gln Arg Arg Ile
       130                 135                 140
   Thr Asp Ala Leu Tyr Asp Arg Asp Asp Ala Leu Glu Asp Arg Val Glu
   145                 150                 155                 160
   Asn Tyr His Pro Gly Asp Thr Asp Met Thr Trp Ala Arg Leu Thr Gln
                   165                 170                 175
   Trp Arg Gly Leu Val Ala Ser Ser Leu Asp His Pro Pro His Ser Glu
               180                 185                 190
   Ile Thr Ser Val Arg Leu Thr Gly Ala Ser Gly Ser Thr Ser Val Asp
           195                 200                 205
   Leu Ala Ala Gly Trp Leu Ala Arg Arg Leu Asn Val Pro Val Ile Arg
       210                 215                 220
   Glu Val Thr Asp Ala Pro Thr Val Ser Thr Asp Glu Phe Gly Thr Pro
   225                 230                 235                 240
   Leu Leu Ala Ile Gln Arg Leu Glu Ile Val Arg Thr Thr Gly Ser Ile
                   245                 250                 255
   Ile Ile Thr Ile His Asp Ala His Thr Leu Gln Val Glu Met Pro Glu
               260                 265                 270
   Ser Gly Asn Ala Pro Ser Leu Val Ala Ile Gly Arg Arg Ser Glu Ser
           275                 280                 285
   Asp Cys Leu Ser Glu Glu Leu Arg His Met Asp Pro Asp Leu Gly Tyr
       290                 295                 300
   Gln His Ala Leu Ser Gly Leu Ser Ser Val Lys Leu Glu Thr Val
   305                 310                 315
<210> 7
   <211> 960
   <212> DNA
   <213> Corynebacterium glutamicum
<220>
   <221> CDS
   <222> (1)..(957)
   <223> opcA-Allel
<220>
   <221> mutation
   <222> (319) .. (319)
   <223> T → C Transition
<220>
   <221> mutation
   <222> (402)..(402)
   <223> C → T Transition
<220>
   <221> mutation
   <222> (600)..(600)
   <223> T → C Transition
<220>
   <221> mutation
   <222> (648)..(648)
   <223> G → C Transversion
<220>
   <221> mutation
   <222> (657) .. (657)
   <223> A → C Transversion
<220>
   <221> mutation
   <222> (697) .. (697)
   <223> C → T Transition
<220>
   <221> mutation
   <222> (781) .. (781)
   <223> T → C Transition
<400> 7
   atg atc ttt gaa ctt ccg gat acc acc acc cag caa att tcc aag acc        48
   Met Ile Phe Glu Leu Pro Asp Thr Thr Thr Gln Gln Ile Ser Lys Thr
   1               5                   10                  15
   cta act cga ctg cgt gaa tcg ggc acc cag gtc acc acc ggc cga gtg        96
   Leu Thr Arg Leu Arg Glu Ser Gly Thr Gln Val Thr Thr Gly Arg Val
               20                  25                  30
   ctc acc ctc atc gtg gtc act gac tcc gaa agc gat gtc gct gca gtt       144
   Leu Thr Leu Ile Val Val Thr Asp Ser Glu Ser Asp Val Ala Ala Val
           35                  40                  45
   acc gag tcc acc aat gaa gcc tcg cgc gag cac cca tct cgc gtg atc       192
   Thr Glu Ser Thr Asn Glu Ala Ser Arg Glu His Pro Ser Arg Val Ile
       50                  55                  60
   att ttg gtg gtt ggc gat aaa act gca gaa aac aaa gtt gac gca gaa       240
   Ile Leu Val Val Gly Asp Lys Thr Ala Glu Asn Lys Val Asp Ala Glu
   65                  70                  75                  80
   gtc cgt atc ggt ggc gac gct ggt gct tcc gag atg atc atc atg cat       288
   Val Arg Ile Gly Gly Asp Ala Gly Ala Ser Glu Met Ile Ile Met His
                   85                  90                  95
   ctc aac gga cct gtc gct gac aag ctc cag cat gtc gtc aca cca ctg       336
   Leu Asn Gly Pro Val Ala Asp Lys Leu Gln His Val Val Thr Pro Leu
               100                 105                 110
   ttg ctt cct gac acc ccc atc gtt gct tgg tgg cca ggt gaa tca cca       384
   Leu Leu Pro Asp Thr Pro Ile Val Ala Trp Trp Pro Gly Glu Ser Pro
           115                 120                 125
   aag aat cct tcc cag gat cca att gga cgc atc gca caa cga cgc atc       432
   Lys Asn Pro Ser Gln Asp Pro Ile Gly Arg Ile Ala Gln Arg Arg Ile
       130                 135                 140
   act gat gct ttg tac gac cgt gat gac gca cta gaa gat cgt gtt gag       480
   Thr Asp Ala Leu Tyr Asp Arg Asp Asp Ala Leu Glu Asp Arg Val Glu
   145                 150                 155                 160
   aac tat cac cca ggt gat acc gac atg acg tgg gcg cgc ctt acc cag       528
   Asn Tyr His Pro Gly Asp Thr Asp Met Thr Trp Ala Arg Leu Thr Gln
                   165                 170                 175
   tgg cgg gga ctt gtt gcc tcc tca ttg gat cac cca cca cac agc gaa       576
   Trp Arg Gly Leu Val Ala Ser Ser Leu Asp His Pro Pro His Ser Glu
               180                 185                 190
   atc act tcc gtg agg ctg acc ggc gca agc ggc agt acc tcg gtg gat       624
   Ile Thr Ser Val Arg Leu Thr Gly Ala Ser Gly Ser Thr Ser Val Asp
           195                 200                 205
   ttg gct gca ggc tgg ttg gcg cgc agg ctg aac gtg cct gtg atc cgc       672
   Leu Ala Ala Gly Trp Leu Ala Arg Arg Leu Asn Val Pro Val Ile Arg
       210                 215                 220
   gag gtg aca gat gct ccc acc gtg tca acc gat gag ttt ggt act cca       720
   Glu Val Thr Asp Ala Pro Thr Val Ser Thr Asp Glu Phe Gly Thr Pro
   225                 230                 235                 240
   ctg ctg gct atc cag cgc ctg gag atc gtt cgc acc acc ggc tcg atc       768
   Leu Leu Ala Ile Gln Arg Leu Glu Ile Val Arg Thr Thr Gly Ser Ile
                   245                 250                 255
   atc atc acc atc cat gac gct cat acc ctt cag gta gag atg ccg gaa       816
   Ile Ile Thr Ile His Asp Ala His Thr Leu Gln Val Glu Met Pro Glu
               260                 265                 270
   tcc ggc aat gcc cca tcg ctg gtg gct att ggt cgt cga agt gag tcc       864
   Ser Gly Asn Ala Pro Ser Leu Val Ala Ile Gly Arg Arg Ser Glu Ser
           275                 280                 285
   gac tgc ttg tct gag gag ctt cgc cac atg gat cca gat ttg ggc tac       912
   Asp Cys Leu Ser Glu Glu Leu Arg His Met Asp Pro Asp Leu Gly Tyr
       290                 295                 300
   cag cac gca cta tcc ggc ttg tcc agc gtc aag ctg gaa acc gtc taa       960
   Gln His Ala Leu Ser Gly Leu Ser Ser Val Lys Leu Glu Thr Val
   305                 310                 315
<210> 8
   <211> 319
   <212> PRT
   <213> Corynebacterium glutamicum
<400> 8
   Met Ile Phe Glu Leu Pro Asp Thr Thr Thr Gln Gln Ile Ser Lys Thr
   1               5                   10                  15
   Leu Thr Arg Leu Arg Glu Ser Gly Thr Gln Val Thr Thr Gly Arg Val
               20                  25                  30
   Leu Thr Leu Ile Val Val Thr Asp Ser Glu Ser Asp Val Ala Ala Val
   35 40 45
   Thr Glu Ser Thr Asn Glu Ala Ser Arg Glu His Pro Ser Arg Val Ile
   50 55 60
   Ile Leu Val Val Gly Asp Lys Thr Ala Glu Asn Lys Val Asp Ala Glu
   65 70 75 80
   Val Arg Ile Gly Gly Asp Ala Gly Ala Ser Glu Met Ile Ile Met His
   85 90 95
   Leu Asn Gly Pro Val Ala Asp Lys Leu Gln His Val Val Thr Pro Leu
   100 105 110
   Leu Leu Pro Asp Thr Pro Ile Val Ala Trp Trp Pro Gly Glu Ser Pro
   115 120 125
   Lys Asn Pro Ser Gln Asp Pro Ile Gly Arg Ile Ala Gln Arg Arg Ile
   130 135 140
   Thr Asp Ala Leu Tyr Asp Arg Asp Asp Ala Leu Glu Asp Arg Val Glu
   145 150 155 160
   Asn Tyr His Pro Gly Asp Thr Asp Met Thr Trp Ala Arg Leu Thr Gln
   165 170 175
   Trp Arg Gly Leu Val Ala Ser Ser Leu Asp His Pro Pro His Ser Glu
   180 185 190
   Ile Thr Ser Val Arg Leu Thr Gly Ala Ser Gly Ser Thr Ser Val Asp
   195 200 205
   Leu Ala Ala Gly Trp Leu Ala Arg Arg Leu Asn Val Pro Val Ile Arg
   210 215 220
   Glu Val Thr Asp Ala Pro Thr Val Ser Thr Asp Glu Phe Gly Thr Pro
   225 230 235 240
   Leu Leu Ala Ile Gln Arg Leu Glu Ile Val Arg Thr Thr Gly Ser Ile
   245 250 255
   Ile Ile Thr Ile His Asp Ala His Thr Leu Gln Val Glu Met Pro Glu
   260 265 270
   Ser Gly Asn Ala Pro Ser Leu Val Ala Ile Gly Arg Arg Ser Glu Ser
   275 280 285
   Asp Cys Leu Ser Glu Glu Leu Arg His Met Asp Pro Asp Leu Gly Tyr
       290                 295                 300
   Gln His Ala Leu Ser Gly Leu Ser Ser Val Lys Leu Glu Thr Val
   305                 310                 315
<210> 9
   <211> 1600
   <212> DNA
   <213> Corynebacterium glutamicum
<220>
   <221> CDS
   <222> (335)..(1291)
   <223> opcA-Allel
<220>
   <221> mutation
   <222> (653)..(653)
   <223> T → C Transition
<220>
   <221> mutation
   <222> (736) .. (736)
   <223> C → T Transition
<220>
   <221> mutation
   <222> (934) .. (934)
   <223> T → C Transition
<220>
   <221> mutation
   <222> (982)..(982)
   <223> G → C Transversion
<220>
   <221> mutation
   <222> (991) .. (991)
   <223> A → C Transversion
<220>
   <221> mutation
   <222> (1031) .. (1031)
   <223> C → T Transition
<220>
   <221> mutation
   <222> (1115) .. (1115)
   <223> T → C Transition
<400> 9
   gtgtgctcat ccgcttcggt tccaaggttc caggttctgc catggaagtc cgtgacgtca     60
   acatggactt ctcctactca gaatccttca ctgaagaatc acctgaagca tacgagcgcc    120
   tcattttgga tgcgctgtta gatgaatcca gcctcttccc taccaacgag gaagtggaac    180
   tgagctggaa gattctggat ccaattcttg aagcatggga tgccgatgga gaaccagagg    240
   attacccagc gggtacgtgg ggtccaaaga gcgctgatga aatgctttcc cgcaacggtc    300
   acacctggcg caggccataa tttaggggca aaaa atg atc ttt gaa ctt ccg gat    355
                                         Met Ile Phe Glu Leu Pro Asp
                                         1               5
   acc acc acc cag caa att tcc aag acc cta act cga ctg cgt gaa tcg      403
   Thr Thr Thr Gln Gln Ile Ser Lys Thr Leu Thr Arg Leu Arg Glu Ser
           10                  15                  20
   ggc acc cag gtc acc acc ggc cga gtg ctc acc ctc atc gtg gtc act      451
   Gly Thr Gln Val Thr Thr Gly Arg Val Leu Thr Leu Ile Val Val Thr
       25                  30                  35
   gac tcc gaa agc gat gtc gct gca gtt acc gag tcc acc aat gaa gcc      499
   Asp Ser Glu Ser Asp Val Ala Ala Val Thr Glu Ser Thr Asn Glu Ala
   40                  45                  50                  55
   tcg cgc gag cac cca tct cgc gtg atc att ttg gtg gtt ggc gat aaa      547
   Ser Arg Glu His Pro Ser Arg Val Ile Ile Leu Val Val Gly Asp Lys
                   60                  65                  70
   act gca gaa aac aaa gtt gac gca gaa gtc cgt atc ggt ggc gac gct      595
   Thr Ala Glu Asn Lys Val Asp Ala Glu Val Arg Ile Gly Gly Asp Ala
               75                  80                  85
   ggt gct tcc gag atg atc atc atg cat ctc aac gga cct gtc gct gac      643
   Gly Ala Ser Glu Met Ile Ile Met His Leu Asn Gly Pro Val Ala Asp
           90                  95                  100
   aag ctc cag cat gtc gtc aca cca ctg ttg ctt cct gac acc ccc atc      691
   Lys Leu Gln His Val Val Thr Pro Leu Leu Leu Pro Asp Thr Pro Ile
       105                 110                 115
   gtt gct tgg tgg cca ggt gaa tca cca aag aat cct tcc cag gat cca      739
   Val Ala Trp Trp Pro Gly Glu Ser Pro Lys Asn Pro Ser Gln Asp Pro
   120                 125                 130                 135
   att gga cgc atc gca caa cga cgc atc act gat gct ttg tac gac cgt      787
   Ile Gly Arg Ile Ala Gln Arg Arg Ile Thr Asp Ala Leu Tyr Asp Arg
                   140                 145                 150
   gat gac gca cta gaa gat cgt gtt gag aac tat cac cca ggt gat acc      835
   Asp Asp Ala Leu Glu Asp Arg Val Glu Asn Tyr His Pro Gly Asp Thr
               155                 160                 165
   gac atg acg tgg gcg cgc ctt acc cag tgg cgg gga ctt gtt gcc tcc      883
   Asp Met Thr Trp Ala Arg Leu Thr Gln Trp Arg Gly Leu Val Ala Ser
           170                 175                 180
   tca ttg gat cac cca cca cac agc gaa atc act tcc gtg agg ctg acc      931
   Ser Leu Asp His Pro Pro His Ser Glu Ile Thr Ser Val Arg Leu Thr
       185                 190                 195
   ggc gca agc ggc agt acc tcg gtg gat ttg gct gca ggc tgg ttg gcg      979
   Gly Ala Ser Gly Ser Thr Ser Val Asp Leu Ala Ala Gly Trp Leu Ala
   200                 205                 210                 215
   cgc agg ctg aac gtg cct gtg atc cgc gag gtg aca gat gct ccc acc      1027
   Arg Arg Leu Asn Val Pro Val Ile Arg Glu Val Thr Asp Ala Pro Thr
                   220                 225                 230
   gtg tca acc gat gag ttt ggt act cca ctg ctg gct atc cag cgc ctg      1075
   Val Ser Thr Asp Glu Phe Gly Thr Pro Leu Leu Ala Ile Gln Arg Leu
               235                 240                 245
   gag atc gtt cgc acc acc ggc tcg atc atc atc acc atc cat gac gct      1123
   Glu Ile Val Arg Thr Thr Gly Ser Ile Ile Ile Thr Ile His Asp Ala
           250                 255                 260
   cat acc ctt cag gta gag atg ccg gaa tcc ggc aat gcc cca tcg ctg      1171
   His Thr Leu Gln Val Glu Met Pro Glu Ser Gly Asn Ala Pro Ser Leu
       265                 270                 275
   gtg gct att ggt cgt cga agt gag tcc gac tgc ttg tct gag gag ctt      1219
   Val Ala Ile Gly Arg Arg Ser Glu Ser Asp Cys Leu Ser Glu Glu Leu
   280                 285                 290                 295
   cgc cac atg gat cca gat ttg ggc tac cag cac gca cta tcc ggc ttg      1267
   Arg His Met Asp Pro Asp Leu Gly Tyr Gln His Ala Leu Ser Gly Leu
                   300                 305                 310
   tcc agc gtc aag ctg gaa acc gtc taaggagaaa tacaacacta tggttgatgt     1321
   Ser Ser Val Lys Leu Glu Thr Val
               315
   agtacgcgca cgcgatactg aagatttggt tgcacaggct gcctccaaat tcattgaggt   1381
   tgttgaagca gcaactgcca ataatggcac cgcacaggta gtgctcaccg gtggtggcgc   1441
   cggcatcaag ttgctggaaa agctcagcgt tgatgcggct gaccttgcct gggatcgcat   1501
   tcatgtgttc ttcggcgatg agcgcaatgt ccctgtcagt gattctgagt ccaatgaggg   1561
   ccaggctcgt gaggcactgt tgtccaaggt ttctatccc                          1600
<210> 10
   <211> 319
   <212> PRT
   <213> Corynebacterium glutamicum
<400> 10
   Met Ile Phe Glu Leu Pro Asp Thr Thr Thr Gln Gln Ile Ser Lys Thr
   1               5                   10                  15
   Leu Thr Arg Leu Arg Glu Ser Gly Thr Gln Val Thr Thr Gly Arg Val
               20                  25                  30
   Leu Thr Leu Ile Val Val Thr Asp Ser Glu Ser Asp Val Ala Ala Val
           35                  40                  45
   Thr Glu Ser Thr Asn Glu Ala Ser Arg Glu His Pro Ser Arg Val Ile
       50                  55                  60
   Ile Leu Val Val Gly Asp Lys Thr Ala Glu Asn Lys Val Asp Ala Glu
   65 70 75 80
   Val Arg Ile Gly Gly Asp Ala Gly Ala Ser Glu Met Ile Ile Met His
   85 90 95
   Leu Asn Gly Pro Val Ala Asp Lys Leu Gln His Val Val Thr Pro Leu
   100 105 110
   Leu Leu Pro Asp Thr Pro Ile Val Ala Trp Trp Pro Gly Glu Ser Pro
   115 120 125
   Lys Asn Pro Ser Gln Asp Pro Ile Gly Arg Ile Ala Gln Arg Arg Ile
   130 135 140
   Thr Asp Ala Leu Tyr Asp Arg Asp Asp Ala Leu Glu Asp Arg Val Glu
   145 150 155 160
   Asn Tyr His Pro Gly Asp Thr Asp Met Thr Trp Ala Arg Leu Thr Gln
   165 170 175
   Trp Arg Gly Leu Val Ala Ser Ser Leu Asp His Pro Pro His Ser Glu
   180 185 190
   Ile Thr Ser Val Arg Leu Thr Gly Ala Ser Gly Ser Thr Ser Val Asp
   195 200 205
   Leu Ala Ala Gly Trp Leu Ala Arg Arg Leu Asn Val Pro Val Ile Arg
   210 215 220
   Glu Val Thr Asp Ala Pro Thr Val Ser Thr Asp Glu Phe Gly Thr Pro
   225 230 235 240
   Leu Leu Ala Ile Gln Arg Leu Glu Ile Val Arg Thr Thr Gly Ser Ile
   245 250 255
   Ile Ile Thr Ile His Asp Ala His Thr Leu Gln Val Glu Met Pro Glu
   260 265 270
   Ser Gly Asn Ala Pro Ser Leu Val Ala Ile Gly Arg Arg Ser Glu Ser
   275 280 285
   Asp Cys Leu Ser Glu Glu Leu Arg His Met Asp Pro Asp Leu Gly Tyr
   290 295 300
   Gln His Ala Leu Ser Gly Leu Ser Ser Val Lys Leu Glu Thr Val
   305 310 315
<210> 11
   <211> 20
   <212> DNA
   <213> Corynebacterium glutamicum
<220>
   <221> misc_feature
   <222> (1)..(20)
   <223> Primer opcA-A1
<400> 11
   cacctggcgc aggccataat 20
<210> 12
   <211> 20
   <212> DNA
   <213> Corynebacterium glutamicum
<220>
   <221> misc_feature
   <222> (1)..(20)
   <223> Primer opcA-E1
<400> 12
   cgcgtgcgcg tactacatca 20
<210> 13
   <211> 20
   <212> DNA
   <213> Corynebacterium glutamicum
<220>
   <221> misc_feature
   <222> (1) .. (20)
   <223> Primer opcA-int1
<400> 13
   acggacctgt cgctgacaag 20
<210> 14
   <211> 20
   <212> DNA
   <213> Corynebacterium glutamicum
<220>
   <221> misc_feature
   <222> (1)..(20)
   <223> Primer opcA-int2
<400> 14
   cggattccgg catctctacc 20
<210> 15
   <211> 519
   <212> DNA
   <213> Corynebacterium glutamicum
<220>
   <221> CDS
   <222> (1) .. (519)
   <223> Leseraster
<220>
   <221> mutation
   <222> (28)..(28)
   <223> C an Position 28 entspricht C an Position 319 von SEQ ID NO:5
<220>
   <221> mutation
   <222> (366)..(366)
   <223> C an Position 366 enspricht C an Position 657 von SEQ ID NO:5
<220>
   <221> mutation
   <222> (406)..(406)
   <223> T an Position 406 entspricht T an Position 697 von SEQ ID NO:5
<220>
   <221> mutation
   <222> (490)..(490)
   <223> C an Position 490 entspricht C an Position 781 von SEQ ID NO:5
<400> 15
   aac gga cct gtc gct gac aag ctc cag cat gtc gtc aca cca ctg ttg       48
   Asn Gly Pro Val Ala Asp Lys Leu Gln His Val Val Thr Pro Leu Leu
   1               5                   10                  15
   ctt cct gac acc ccc atc gtt gct tgg tgg cca ggt gaa tca cca aag       96
   Leu Pro Asp Thr Pro Ile Val Ala Trp Trp Pro Gly Glu Ser Pro Lys
               20                  25                  30
   aat cct tcc cag gac cca att gga cgc atc gca caa cga cgc atc act      144
   Asn Pro Ser Gln Asp Pro Ile Gly Arg Ile Ala Gln Arg Arg Ile Thr
           35                  40                  45
   gat gct ttg tac gac cgt gat gac gca cta gaa gat cgt gtt gag aac      192
   Asp Ala Leu Tyr Asp Arg Asp Asp Ala Leu Glu Asp Arg Val Glu Asn
       50                  55                  60
   tat cac cca ggt gat acc gac atg acg tgg gcg cgc ctt acc cag tgg      240
   Tyr His Pro Gly Asp Thr Asp Met Thr Trp Ala Arg Leu Thr Gln Trp
   65                  70                  75                  80
   cgg gga ctt gtt gcc tcc tca ttg gat cac cca cca cac agc gaa atc      288
   Arg Gly Leu Val Ala Ser Ser Leu Asp His Pro Pro His Ser Glu Ile
                   85                  90                  95
   act tcc gtg agg ctg acc ggt gca agc ggc agt acc tcg gtg gat ttg      336
   Thr Ser Val Arg Leu Thr Gly Ala Ser Gly Ser Thr Ser Val Asp Leu
               100                 105                 110
   gct gca ggc tgg ttg gcg cgg agg ctg aac gtg cct gtg atc cgc gag        384
   Ala Ala Gly Trp Leu Ala Arg Arg Leu Asn Val Pro Val Ile Arg Glu
       115                 120                 125
   gtg aca gat gct ccc acc gtg tca acc gat gag ttt ggt act cca ctg        432
   Val Thr Asp Ala Pro Thr Val Ser Thr Asp Glu Phe Gly Thr Pro Leu
       130                 135                 140
   ctg gct atc cag cgc ctg gag atc gtt cgc acc acc ggc tcg atc atc        480
   Leu Ala Ile Gln Arg Leu Glu Ile Val Arg Thr Thr Gly Ser Ile Ile
   145                 150                 155                 160
   atc acc atc cat gac gct cat acc ctt cag gta gag atg                    519
   Ile Thr Ile His Asp Ala His Thr Leu Gln Val Glu Met
                   165                 170
<210> 16
   <211> 173
   <212> PRT
   <213> Corynebacterium glutamicum
<400> 16
   Asn Gly Pro Val Ala Asp Lys Leu Gln His Val Val Thr Pro Leu Leu
   1               5                   10                  15
   Leu Pro Asp Thr Pro Ile Val Ala Trp Trp Pro Gly Glu Ser Pro Lys
               20                  25                  30
   Asn Pro Ser Gln Asp Pro Ile Gly Arg Ile Ala Gln Arg Arg Ile Thr
           35                  40                  45
   Asp Ala Leu Tyr Asp Arg Asp Asp Ala Leu Glu Asp Arg Val Glu Asn
       50                  55                  60
   Tyr His Pro Gly Asp Thr Asp Met Thr Trp Ala Arg Leu Thr Gln Trp
   65                  70                  75                  80
   Arg Gly Leu Val Ala Ser Ser Leu Asp His Pro Pro His Ser Glu Ile
                   85                  90                  95
   Thr Ser Val Arg Leu Thr Gly Ala Ser Gly Ser Thr Ser Val Asp Leu
               100                 105                 110
   Ala Ala Gly Trp Leu Ala Arg Arg Leu Asn Val Pro Val Ile Arg Glu
           115                 120                 125
   Val Thr Asp Ala Pro Thr Val Ser Thr Asp Glu Phe Gly Thr Pro Leu
       130                 135                 140
   Leu Ala Ile Gln Arg Leu Glu Ile Val Arg Thr Thr Gly Ser Ile Ile
   145                 150                 155                 160
   Ile Thr Ile His Asp Ala His Thr Leu Gln Val Glu Met
                   165                 170
<210> 17
   <211> 519
   <212> DNA
   <213> Corynebacterium glutamicum
<220>
   <221> CDS
   <222> (1)..(519)
   <223> Leseraster
<220>
   <221> mutation
   <222> (28) .. (28)
   <223> C an Position 28 entspricht C an Position 319 von SEQ ID NO:7
<220>
   <221> mutation
   <222> (111)..(111)
   <223> T an Position 111 entspricht T an Position 402 von SEQ ID NO:7.
<220>
   <221> mutation
   <222> (309)..(309)
   <223> C an Position 309 entspricht C an Position 600 von SEQ ID NO:7
<220>
   <221> mutation
   <222> (357)..(357)
   <223> C an Position 357 entspricht C an Position 648 von SEQ ID NO:7
<220>
   <221> mutation
   <222> (366)..(366)
   <223> C an Position 366 entspricht C an Position 657 von SEQ ID NO:7
<220>
   <221> mutation
   <222> (406)..(406)
   <223> T an Position 406 entspricht T an Position 697 von SEQ ID NO:7
<220>
   <221> mutation
   <222> (490)..(490)
   <223> C an Position 490 entspricht C an Position 781 von SEQ ID NO:7
<400> 17
   aac gga cct gtc gct gac aag ctc cag cat gtc gtc aca cca ctg ttg       48
   Asn Gly Pro Val Ala Asp Lys Leu Gln His Val Val Thr Pro Leu Leu
   1               5                   10                  15
   ctt cct gac acc ccc atc gtt gct tgg tgg cca ggt gaa tca cca aag       96
   Leu Pro Asp Thr Pro Ile Val Ala Trp Trp Pro Gly Glu Ser Pro Lys
               20                  25                  30
   aat cct tcc cag gat cca att gga cgc atc gca caa cga cgc atc act      144
   Asn Pro Ser Gln Asp Pro Ile Gly Arg Ile Ala Gln Arg Arg Ile Thr
           35                  40                  45
   gat gct ttg tac gac cgt gat gac gca cta gaa gat cgt gtt gag aac      192
   Asp Ala Leu Tyr Asp Arg Asp Asp Ala Leu Glu Asp Arg Val Glu Asn
       50                  55                  60
   tat cac cca ggt gat acc gac atg acg tgg gcg cgc ctt acc cag tgg      240
   Tyr His Pro Gly Asp Thr Asp Met Thr Trp Ala Arg Leu Thr Gln Trp
   65                  70                  75                  80
   cgg gga ctt gtt gcc tcc tca ttg gat cac cca cca cac agc gaa atc      288
   Arg Gly Leu Val Ala Ser Ser Leu Asp His Pro Pro His Ser Glu Ile
                   85                  90                  95
   act tcc gtg agg ctg acc ggc gca agc ggc agt acc tcg gtg gat ttg      336
   Thr Ser Val Arg Leu Thr Gly Ala Ser Gly Ser Thr Ser Val Asp Leu
               100                 105                 110
   gct gca ggc tgg ttg gcg cgc agg ctg aac gtg cct gtg atc cgc gag      384
   Ala Ala Gly Trp Leu Ala Arg Arg Leu Asn Val Pro Val Ile Arg Glu
           115                 120                 125
   gtg aca gat gct ccc acc gtg tca acc gat gag ttt ggt act cca ctg      432
   Val Thr Asp Ala Pro Thr Val Ser Thr Asp Glu Phe Gly Thr Pro Leu
       130                 135                 140
   ctg gct atc cag cgc ctg gag atc gtt cgc acc acc ggc tcg atc atc      480
   Leu Ala Ile Gln Arg Leu Glu Ile Val Arg Thr Thr Gly Ser Ile Ile
   145                 150                 155                 160
   atc acc atc cat gac gct cat acc ctt cag gta gag atg                  519
   Ile Thr Ile His Asp Ala His Thr Leu Gln Val Glu Met
                   165                 170
<210> 18
   <211> 173
   <212> PRT
   <213> Corynebacterium glutamicum
<400> 18
   Asn Gly Pro Val Ala Asp Lys Leu Gln His Val Val Thr Pro Leu Leu
   1               5                   10                  15
   Leu Pro Asp Thr Pro Ile Val Ala Trp Trp Pro Gly Glu Ser Pro Lys
               20                  25                  30
   Asn Pro Ser Gln Asp Pro Ile Gly Arg Ile Ala Gln Arg Arg Ile Thr
           35                  40                  45
   Asp Ala Leu Tyr Asp Arg Asp Asp Ala Leu Glu Asp Arg Val Glu Asn
       50                  55                  60
   Tyr His Pro Gly Asp Thr Asp Met Thr Trp Ala Arg Leu Thr Gln Trp
   65                  70                  75                  80
   Arg Gly Leu Val Ala Ser Ser Leu Asp His Pro Pro His Ser Glu Ile
                   85                  90                  95
   Thr Ser Val Arg Leu Thr Gly Ala Ser Gly Ser Thr Ser Val Asp Leu
               100                 105                 110
   Ala Ala Gly Trp Leu Ala Arg Arg Leu Asn Val Pro Val Ile Arg Glu
           115                 120                 125
   Val Thr Asp Ala Pro Thr Val Ser Thr Asp Glu Phe Gly Thr Pro Leu
       130                 135                 140
   Leu Ala Ile Gln Arg Leu Glu Ile Val Arg Thr Thr Gly Ser Ile Ile
   145                 150                 155                 160
   Ile Thr Ile His Asp Ala His Thr Leu Gln Val Glu Met
                   165                 170
<210> 19
   <211> 1263
   <212> DNA
   <213> Corynebacterium glutamicum
<220>
   <221> CDS
   <222> (1)..(1263)
   <223> lysC-Wildtypgen
<400> 19
   gtg gcc ctg gtc gta cag aaa tat ggc ggt tcc tcg ctt gag agt gcg        48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct        96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat       144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt       192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc       240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg       288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc      336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc      384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc      432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg      480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt      528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct      1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg      1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt      1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca      1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat      1248
   Leu His Glu Gln Phe Gln Leu Gly Gly Glu Asp Glu Ala Val Val Tyr
                   405                 410                 415
   gca ggc acc gga cgc                                                  1263
   Ala Gly Thr Gly Arg
               420
<210> 20
   <211> 421
   <212> PRT
   <213> Corynebacterium glutamicum
<400> 20
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg

## Beschreibung

'Gegenstand der Erfindung sind Mutanten und Allele des opcA-Gens kodierend für Varianten der OpcA-Untereinheit der Glucose-6-Phosphat-Dehydrogenase (EC: 1.1.1.49) und Verfahren zur Herstellung von Aminosäuren, insbesondere L-Lysin und L-Tryptophan unter Verwendung von Bakterien, die diese Allele enthalten.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet.. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht. Eine zusammenfassende Darstellung über verschiedenste Aspekte der Genetik, des Stoffwechsels und der Biotechnologie von Corynebacterium glutamicum findet sich bei Pühler (chief ed.) im Journal of Biotechnology 104 (1-3), 1-338, 2003.

Moritz et al.. (European Journal of Biochemistry 267, 3442-3452 (2000)) berichten über physiologische und biochemische Untersuchungen an der Glucose-6-Phosphat-Dehydrogenase von Corynebacterium glutamicum. Nach Untersuchungen von Moritz et al. besteht die Glucose-6-phosphat-Dehydrogenase aus einer Zwf-Untereinheit und.einer OpcA-Untereinheit.

Eine Methode zur Bestimmung der enzymatischen Aktivität der Glucose-6-Phosphat-Dehydrogenase ist bei Moritz et al. (European Journal of Biochemistry 267, 3442-3452 (2000) beschrieben.

Die Nukleotidsequenz des für die OpcA-Unfereinheit der Glucose-6-Phosphat-Dehydrogenase von Corynebacterium glutamicum kodierenden Gens ist unter anderem in der Datenbank des National Center for Biotechnology Information (NCBI, Bethesda, MD, USA) der National Library of Medicin (Bethesda, MD, USA) unter der Nummer AX121828 allgemein verfügbar.. Sie kann weiterhin der Patentanmeldung EP1108790 als Sequenz Nr. 1744 entnommen werden.

Weitere Datenbanken wie beispielsweise die Nukleotidsequenz-Datenbank der European Molecular Biologie Laboratories (EMBL, Heidelberg, Deutschland bzw. Cambridge, UK) oder die des Swiss Institute of Bioinformatics (Swissprot, Genève, Switzerland) oder die der Protein Information Resource Database (PIR, Washington, DC, USA) können ebenfalls genutzt werden.

Die mikrobielle Biosynthese von L-Aminosäuren in coryneformen Bakterien ist ein komplexes System und vielschichtig mit diversen anderen Stoffwechselwegen in der Zelle vernetzt. Daher kann keine Vorhersage gemacht werden, ob durch Mutation das OpcA-Polypeptid der Glucose-6-Phosphat-Dehydrogenase dergestalt verändert, dass die Produktion von L-Aminosäuren verbessert wird. Der besseren Übersichtlichkeit halber ist die Nukleotidsequenz der für die OpcA-Untereinheit der Glucose-6-Phosphat-Dehydrogenase aus Corynebacterium glutamicum kodierenden zwf-Gens ("Wildtyp-Gen") gemäß den Angaben der NCBI-Datenbarik in SEQ ID NO:1 und die sich daraus ergebende Aminosäuresequenz der kodierten Glucose-6-Phosphat-Dehydrogenase in SEQ ID NO:2 und 4 dargestellt. In SEQ ID NO:3 sind stromaufwärts (upstream) und stromabwärts (downstream) gelegene Nukleotidsequenzen zusätzlich angegeben.

### Aufgabe der Erfindung

Die Erfinder haben sich zur Aufgabe gestellt, verbesserte Stämme von Mikroorganismen bereitzustellen, die erhöhte. Mengen von Aminosäuren, insbesondere L-Lysin und L-Tryptophan, prodtizieren.

### Beschreibung der Erfindung

Gegenstand der Erfindung sind durch in vitro und/oder in vivo erzeugte, beziehungsweise isolierte. Mutanten coryneformer Bakterien, die bevorzugt Aminosäuren ausscheiden, und welche ein Gen bzw. Allel enthalten, das für die OpcA-Untereinheit der Glucose-6-Phosphat-Dehydrogenase kodiert, wobei die Aminosäuresequenz des Polypeptids ausgewählt ist aus der Gruppe, bestehend aus
a) SEQ ID NO: 6, in der sich
   an Position 107 der Aminosäuresequenz L-Histidin
   an Position 219 L-Asparagin,
   an Position 233 L-Serin und
   an Position 261 L-Histidin befinden,
b) SEQ ID NO: 6, wobei sich über die genannten Mutationen hinaus maximal 5 konservative Aminosäureaustausche finden, und
c) SEQ ID NO: 6, wobei sich über genannten Mutationen hinaus maximal 5 Insertionen oder Deletionen finden.

Das Polypeptid, das in den erfindungsgemäßen Mutanten enthalten ist, kann ebenfalls als OpcA-Polypeptid, OpcA-Polypeptid der Glucose-6-phosphat-Dehydrogenase, als OpcA-Untereinheit der Glucose-6-phosphat-Dehydrogenase, oder als OpcA-Polypeptid Untereinheit bezeichnet werden. In der EP 1 108 790 (siehe SEQ ID NO:1744 in Tabelle 1) wird das OpcA-Polypeptid auch als "glucose -6-phosphate dehydrogenase assembly protein" bezeichnet.

Bei den coryneformen Bakterien wird die Gattung Corynebacterium bevorzugt. Besonders bevorzugt sind Aminosäure ausscheidende Stämme, die auf folgenden Arten beruhen:
Corynebacterium efficiens, wie zum Beispiel der Stamm. DSM44549,
Corynebacterium glutamicum, wie zum Beispiel der Stamm ATCC13032,
Corynebacterium thermoaminogenes wie zum Beispiel der Stamm FERM BP-1539, und
Corynebacterium ammoniagenes, wie zum Beispiel der Stamm ATCC6871,
wobei die Art Corynbacterium glutamicum ganz besonders bevorzugt wird.

Einige Vertreter der Art Corynebacterium glutamicum sind im Stand der Technik auch unter anderen Artbezeicnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869, und
Brevibacterium divaricatum ATCC14020.

Bekannte Vertreter Aminosäure ausscheidender Stämme coryneformer Bakterien sind beispielsweise
die L-Lysin produzierenden Stämmen
Corynebacterium glutamicum DM58-1/pDM6 (= DSM4697) beschrieben in EP 0 358 940,
Corynebacterium glutamicum MH20-22B (= DSM16835) beschrieben in Menkel et al. (Applied and Environmental Microbiology 55 (3), 684-688 (1989)), Corynebacterium glutamicum AHP-3 (= FermBP-7382) beschrieben in EP 1 108 790,
Corynebacterium glutamicum NRRL B-11474 beschrieben in US 4,275,157
Corynebacterium thermoaminogenes AJ12521 (= FERM BP-3304) beschrieben in US 5,250,423,
oder die L-Tryptophan produzierenden Stämme
Corynebacterium glutamicum K76 (=FermBP-1847) beschrieben in US 5,563,052,
Corynebacterium glutamicum BPS13 (=FermBP-1777) beschrieben in US 5,605,818, und
Corynebacterium glutamicum FermBP-3055 beschrieben in US 5,235,940.

Angaben zur taxonomischen Einordnung von Stämmen dieser Gruppe von Bakterien findet man unter anderem bei Seiler (Journal of General Microbiology 129, 1433-1477 (1983)), Kämpfer und Kroppenstedt (Canadian Journal of Microbiology 42, 989-1005 (1996)), Liebl et al (International Journal of Systematic Bacteriology 41, 255-260 (1991)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit-der Bezeichnung "FERM" können vom National Institute of Advanced Industrial Science and Technology (AIST Tsukuba Central 6, 1-1-1 Higashi, Tsukuba Ibaraki, Japan) bezogen werden. Die genannten Stämme von Corynebacterium thermoaminogenes (FERM BP-1539, FERM BP-1540, FERM BP-1541 und FERM BP-1542) sind in der US-A 5,250,434 beschrieben. Stämme mit der Bezeichnung "NRRL" können von der Agricultural Research Service Patent Culture Collection (ARS, Peoria, Illinois, US) bezogen werden.

Unter proteinogene Aminosäuren versteht man die Aminosäuren, die in.natürlichen Proteinen, das heißt in Proteinen von Mikroorganismen, Pflanzen, Tieren und Menschen vorkommen. Hierzu gehören insbesondere L-Aminosäuren, ausgewählt aus' der Gruppe L-Asparaginsäure, L-Asparagin,' L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin.. Zu den L-Aminosäuren gehört ebenfalls das L-Homoserin.

Die erfindungsgemäßen Mutanten scheiden bevorzugt die genannten proteinogen Aminosäuren, insbesondere L-Lysin und L-Tryptophan aus. Der Begriff Aminosäuren umfasst auch deren Salze wie beispielsweise das Lysin-Monohydrochlorid oder Lysin-Sulfat im Falle der Aminosäure L-Lysin.

Gegenstand der Erfindung sind weiterhin Mutanten coryneformer Bakterien, die ein opcA-Allel enthalten, das für ein OpcA-Polypeptid kodiert, das dadurch gekennzeichnet ist, dass die Aminosäuresequenz des Polypeptids aus der SEQ ID NO:6 besteht, in der sich
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin befinden,
wobei das Allel eine Nukleotidsequenz umfasst, die mit der Nukleotidsequenz eines Polynukleotids identisch ist, das durch eine Polymerasekettenreaktion (PCR) unter Verwendung eines Primerpaars erhältlich ist, deren Nukleotidsequenzen jeweils mindestens 15 aufeinanderfolgende Nukleotide umfassen, die aus der Nukleotidsequenz zwischen Position 1 und 334 von SEQ ID NO:3 und aus der komplementären Nukleotidsequenz zwischen Position 1600 und 1292 von SEQ ID NO:3 ausgewählt werden. Ein Beispiel für ein derartiges Primerpaar ist das in SEQ ID NO:11 und SEQ ID NO:12 dargestellte Primerpaar opcA-A1 und opcA-E1. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Beschrieben sind weiterhin Mutanten coryneformer Bakterien, die ein opcA-Allel enthalten, das für ein OpcA-Polypeptid kodiert, das eine Aminosäuresequenz mit einer Länge entsprechend 319 L-Aminosäuren umfas.st, wobei die Aminosäuresequenz des Polypeptids eine oder mehrere der Aminosäuren aufweist, ausgewählt aus der Gruppe:
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin.

Gegenstand der Erfindung sind weiterhin Mutanten coryneformer Bakterien, die ein opcA-Allel enthalten, das für ein OpcA-Polypeptid, das an Position 107 bis 261 der Aminosäuresequenz die Aminosäuresequenz entsprechend Position 107 bis 261 von SEQ ID NO:6 enthält. Bevorzugt enthält die Aminosäuresequenz des kodierten Polypeptids eine Aminosäuresequenz entsprechend Position 98 bis 270 von SEQ ID NO:6 oder Position 83 bis 285 von SEQ ID NO:6 oder Position 63 bis 305 von SEQ ID NO:6 oder Position 38 bis 315 von SEQ ID NO:6 oder Position 8 bis 315 von SEQ ID NO:6 oder Position 2 bis 315 von SEQ ID NO:6 oder Position 2 bis 317 von SEQ ID NO:6 oder Position 2 bis 319 von SEQ ID NO:6. Ganz besonders bevorzugt umfasst die Länge des kodierten Polypeptids 319 Aminosäuren von SEQ ID NO:6

Beschrieben sind weiterhin Mutanten coryneformer Bakterien, die ein opcA-Allel enthalten, das für ein OpcA-Polypeptid kodiert, wobei die Aminosäuresequenz des Polypeptids eine oder mehrere der Aminosäuren aufweist, ausgewählt aus der Gruppe:
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin
und dessen Aminosäuresequenz außerdem zu mindestens 90%, bevorzugt mindestens 92% oder mindestens 94% oder mindestens 96%, und ganz besonders bevorzugt mindestens 97% oder mindestens 98% oder mindestens 99% oder 100 %identisch ist mit den Aminosäuresequenzen von SEQ ID NO: 6 oder 2.

Gegenstand der Erfindung sind weiterhin Mutanten coryneformer Bakterien, die ein opcA-Allel enthalten, das für ein OpcA-Polypeptid kodiert, wobei die Aminosäuresequenz des Polypeptids aus der SEQ ID NO:6 besteht, in der sich
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin befinden
und dessen Nukleotidsequenz außerdem zu mindestens 90%, bevorzugt mindestens 92% oder mindestens 94% oder mindestens 96%, und ganz besonders bevorzugt mindestens 97% oder mindestens 98% oder mindestens 99% oder 100 % identisch ist mit der Nukleotidsequenz von SEQ ID NO:5.

Ein Beispiel für eine Nukleotidsequenz eines opcA-Allels, das mindestens 99% Identität mit der Nukleotidsequenz von SEQ ID NO:5 besitzt, ist in SEQ ID NO:7 gezeigt. Die Nukleotidsequenz dieses opcA-Allels besitzt zusätzlich zu den Nukleotidaustauschen Thymin gegen Cytosin an Position 319, Adenin gegen Cytosin an Position 657, Cytosin gegen Thymin an Position 697 und Thymin gegen Cytosin an Position 781 (Siehe SEQ ID NO:5) die Nukleotidaustausche Cytosin gegen Thymin an der Position 402 Thymin gegen Cytosin an der Position 600, und Guanin gegen Cytosin an der Position 648 (Siehe SEQ ID NO:7). Die Formulierung "Guanin gegen Cytosin an der Position 648" - und vergleichbare Formulierungen - bedeutet, dass die in der Wildtypsequenz der Kodierregion an der Position 648 vorhandene Nukleobase Guanin (Siehe SEQ ID NO:1) durch Cytosin ersetzt worden ist (Siehe SEQ ID NO:7).

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysis und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Gegenstand der Erfindung sind schließlich Mutanten coryneformer Bakterien, die ein opcA-Allel enthalten, das für ein OpcA-Polypeptid kodiert, welches die Aminosäuresequenz von SEQ ID NO:6 umfasst.

Es ist bekannt, dass durch wirtseigene Enzyme, sogenannte Aminovertidasen, das endständige Methionin bei der Proteinsynthese entfernt wird.

Durch derartige Insertionen und Deletionen oder konservative Aminosäureaustausche wird die enzymatische Aktivität der die entsprechende OpcA-Untereinheit enthaltenden Glucose-6-Phosphat-Dehydrogenasen im wesentlichen nicht berührt. "Im wesentlichen nicht berührt" bedeutet, dass sich die enzymatische Aktivität der genannten Varianten um maximal 10%, maximal 7,5%, maximal 5%, maximal 2,5% oder maximal 1% von der Aktivität einer Glucose-6-Phosphat-Dehydrogenase unterscheidet, welche eine OpcA-Polypeptid-Untereinheit mit der Aminosäuresequenz von SEQ ID NO: 2 enthält, wobei diese die Aminosäureaustausche
a) an Position 107 L-Histidin anstelle L-Tyrosin .
b) an Position 219 L-Asparagin anstelle L-Lysin
c) an Position 233 L-Serin anstelle L-Prolin, und
d) an Position 261 L-Histidin anstelle L-Tyrosin
enthält.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG), Ethylmethansulfonat (EMS), 5-Bromuracil, oder ultraviolettes Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al.. (Agricultural and Biological Chemistry 42(4), 745-752 (1978)) oder bei Konicek et al (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten beziehungsweise Zellen entnommen und vermehrt. Vorzugsweise wird in einem weiteren Schritt deren Fähigkeit untersucht, in einer Satzkultur (batch) unter Verwendung eines geeigneten Nährmediums Aminosäuren, bevorzugt L-Lysin oder. L-Tryptophan, auszuscheiden. Geeignete Nährmedien und Testbedingungen sind unter anderem in der US 6,221,636, in der US 5,840,551, in der US 5,770,409, in der US 5,605,818, in der US 5,275,940 und in der US 4,224,409 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428. (2005))beschrieben, können zahlreiche Mutanten in kurzer Zeit untersucht werden. Auf diese Weise werden Mutanten identifiziert, die im Vergleich zum Elternstamm beziehungsweise nicht mutagenisierten Ausgangsstamm vermehrt Aminosäuren in das Nährmedium oder in das Zellinnere ausscheiden. Dazu gehören beispielsweise solche Mutanten, deren Aminosäuresekretion um mindestens 0,5% erhöht ist.

Anschließend wird aus den Mutanten DNA bereitgestellt, beziehungsweise isoliert und mit,Hilfe der Polymerase-Kettenreaktion unter Verwendung von Primerpaaren, die die Amplifizierung des opcA-Gens beziehungsweise der erfindungsgemäßen opcA-Allele oder der erfindungsgemäßen Mutationen an den Positionen 107, 219, 233 und 261 der Aminosäuresequenz des OpcA-Polypeptides erlauben, das entsprechende Polynukleotid synthetisiert.. Vorzugsweise wird die DNA aus solchen Mutanten isoliert, die im Vergleich zum Ausgangsstamm in erhöhtem Maße Aminosäuren ausscheiden oder im Zellinneren anreichern.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 652 von SEQ ID NO:3. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz von Position 1600 und 1118 von SEQ ID NO:3. Ist die Amplifikation der Kodierregion gewünscht, so wird das Primerpaar vorzugsweise aus der Nukleotidsequenz zwischen Position 1 und 334 von SEQ ID NO:3 und aus der komplementären Nukleotidsequenz zwischen Position 1600 und. 1292 von SEQ ID NO:3 ausgewählt. Ist die Amplifikation eines Teils der Kodierregion, wie beispielsweise in SEQ ID NO:15 und 17 dargestellt, erwünscht, so wird das Primerpaar vorzugsweise aus der Nukleotidsequenz zwischen Position 335 und 652 von SEQ ID NO:3 und aus der komplementären Nukleotidsequenz zwischen Position 1291 und 1118 von SEQ ID NO:3 ausgewählt.

Geeignete Primerpaare sind beispielsweise das unter SEQ ID NO:11 und SEQ ID NO:12 wiedergegebene Primerpaar opcA-A1 und opcA-E1 und das unter SEQ ID NO:13 und SEQ ID NO:14 wiedergegebene Primerpaar opcA-int1 und opcA-int2. Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen, wie dem erfindungsgemäßen opcA-Allel, aus vorgelegter, beispielsweise chromosomaler DNA ("template DNA")durch Amplifikation mittels PCR, werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die VentPolymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von. 10 - 100 mM Tris/HCl und 6 - 55 mM KC1 bei pH 7,5 - 9,3 aufweist. Magnesiumchlorid wird in einer Konzentration von 0,5- 10 mM zugesetzt, falls es nicht in dem vom Hersteller gelieferten Puffer enthalten ist...Dem Reaktionsansatz werden weiterhin Desoxynucleosidtriphosphate in einer Konzentration von 0.,1 - 16,6 mM zugesetzt. Die Primer werden im Reaktionsansatz mit einer Endkonzentration von 0,1 -.3 µM vorgelegt und die Template-DNA im optimalen Fall mit 10² bis 10⁵ Kopien. Es können auch 10⁶ bis 10⁷ Kopien eingesetzt werden.. Die entsprechende Polymerase wird dem Reaktionsansatz in einer Menge von 2-5 Units zugegeben. Ein typischer Reaktionsansatz hat ein Volumen von 20 - 100 µl.

Als weitere Zusätze können der Reaktion Rinderserumalbumin, Tween-20, Gelatin, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al.. (Nucleic Acids Research 18 (21): 6409-6412). Anschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR. dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; der Begriff Nukleinsäurefragment ist ebenfalls gebräuchlich.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press, 1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994.).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al.. (Nucleic Acids Research 18, 1067pp (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das. "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren opcA-Allele für OpcA-Polypeptide kodieren, welche die erfindungsgemäßen Mutationen enthalten.

Gegenstand der Erfindung ist dementsprechend eine Mutante eines coryneformen Bakteriums, die durch folgende Schritte erhältlich ist:
a) Behandlung eines coryneformen Bakteriums, das die Fähigkeit besitzt Aminosäuren auszuscheiden, mit einem mutagenen Agent,
b) Isolierung und Vermehrung der in a) erzeugten Mutante,
c) vorzugsweise Bestimmung, der Fähigkeit der Mutante in einem Medium mindestens 0,5% mehr Aminosäure auszuscheiden oder im Zellinneren anzureichern als das in a) eingesetzte coryneforme Bakterium,
d) Bereitstellung von Nukleinsäure aus der in b) erhaltenen Mutante,
e) Herstellung eines Nukleinsäuremoleküls/ Amplifikates/ Nukleinsäurefragments unter Verwendung der Polymerasekettenreaktion, der Nukleinsäure aus d), und eines Primerpaares bestehend aus einem ersten Primer umfassend mindestens 15 aufeinanderfolgenden Nukleotide ausgewählt aus der Nukleotidsequenz zwischen Position 1 und 334 von SEQ ID NO:3 und einem zweitem Primer umfassend mindestens 15 aufeinanderfolgenden Nukleotide ausgewählt aus der komplementären Nukleotidsequenz zwischen Position 1600 und 1292 von SEQ ID-NO:3,
f) Bestimmung der Nukleotidsequenz des in e) erhaltenen Nukleinsäuremoleküls, und Bestimmung der kodierten Aminosäuresequenz,
g) gegebenfalls Vergleich der in f) bestimmen Aminosäuresesequenz mit SEQ ID NO:6, und
h) Identifizierung einer Mutante, die ein Polynukleotid enthält, das für ein Polypeptid kodiert, dessen Aminosäuresequenz aus des SEQ ID NO: 6 besteht, in der sich
   i) an Position 107 der Aminosäuresequenz L-Histidin,
   ii) an Position 219 der Aminosäuresequenz L-Asparagin,
   iii) an Position 233 der Aminosäuresequenz L-Serin, und
   iv) an Position 261 der Aminosäuresequenz L-Histidin befinden.

Die auf diese Weise erzeugten Mutanten enthalten typischerweise eine (1) Kopie eines der beschriebenen opcA Allele.. Das hier beschriebene Verfahren ist ebenfalls Gegenstand der Erfindung.

In der SEQ ID NO:5 ist.beispielhaft die Kodierregionen eines opcA-Allels einer erfindungsgemäßen Mutante wiedergegeben. Die Kodierregion des Wildtypgens ist als SEQ ID NO:1 wiedergegeben.

SEQ ID NO:1 enthält an Position 319 bis 321 das für die Aminosäure L-Tyrosin kodierende TAT Kodon. SEQ ID NO:5 enthält an Position 319 die Nukleobase Cytosin. Durch diese Thymin Cytosin Transition entsteht an Position 319 bis 321 das für die Aminosäure L-Histidin kodierende CAT Kodon.

SEQ ID-NO:1 enthält an Position 655 bis 657 das für die Aminosäure L-Lysin kodierende AAA Kodon. SEQ ID NO:5 enthält an Position 657 die Nukleobase Cytosin. Durch diese Adenin Cytosin Transversion entsteht an Position 655 bis 657 das für die Aminosäure-L-Asparagin kodierende AAC Kodon.

SEQ ID NO:1 enthält an Position 697 bis 699 das für die Aminosäure L-Prolin kodierende CCA Kodon. SEQ ID NO:5 enthält an Position 697 die Nukleobase Thymin.. Durch diese Cytosin Thymin Transition entsteht an Position 697 bis 699 das für die Aminosäure L-Serin kodierende TCA Kodon.

SEQ ID NO:1 enthält an Position 781 bis 783 das für die Aminosäure L-Tyrosin kodierende TAT Kodon. SEQ ID NO:5 enthält an Position 781 die Nukleobase Cytosin. Durch diese Thymin Cytosin Transition entsteht an Position 781 bis 783 das für die Aminosäure L-Histidin kodierende CAT Kodon.

Darüber hinaus kann die in SEQ ID NO: 5 dargestellte Nukleotidsequenz weitere Basenaustausche enthalten, die sich durch die Mutagenesebehandlung ergeben haben, sich jedoch nicht in einer veränderten Aminosäuresequenz äußern.

Derartige Mutationen werden in der Fachwelt auch als stille oder neutrale Mutationen bezeichnet. Diese stillen Mutationen können ebenfalls in dem für die Mutagenesebehandlung eingesetzten coryneformen Bakterium bereits enthalten sein. Beispiele für derartige stille Mutationen sind die Cytosin-Thymin Transition an Position 402, die Thymin-Cytosin Transition an Position 600 und die Guanin-Cytosin Transversion an Position 648 wie in SEQ ID NO:7 dargestellt.

Die für die Mutagenese verwendeten coryneformen Bakterien besitzen bevorzugt bereits die Fähigkeit, die gewünschte Aminosäure in das sie umgebende Nährmedium beziehungsweise Fermentationsbrühe auszuscheiden oder im Zellinneren anzureichern.

L-Lysin produzierende coryneforme Bakterien besitzen typischerweise eine "feed back" resistente beziehungsweise desensibilisierte Aspartatkinase. Unter "feed back" resistenten Aspartatkinasen versteht man Aspartatkinasen, die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene beziehungsweise Allele werden auch als lysC^{FBR}-Allele bezeichnet. Im Stand der Technik (Tabelle 1) sind zahlreiche lysC^{FBR}-Allele beschrieben, die für Aspartatkinase-Varianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:19 und das von diesem Gen kodierte Protein in SEQ ID NO:20 dargestellt.

**Tabelle 1**

| lysC^{FBR}-Allele kodierend für feed back resistente Aspartatkinasen | | | |
|---|---|---|---|
| Bezeichnung des Allels | Weitere Angaben | Referenz | Zugangsnummer |
| lysC^{FBR}-E05108 | | JP 1993184366-A (Sequenz 1) | E05108 |
| lysC^{FBR}-E06825 | lysC A279T | JP 1994062866-A (Sequenz 1) | E06825 |
| lysC^{FBR}-E06826 | lysC A279T | JP 1994062866-A (Sequenz 2) | E06826 |
| lysC^{FBR}-E06827 | | JP 1994062866-A (Sequenz 3) | E06827 |
| lysC^{FBR}-E08177 | | JP 1994261766-A (Sequenz 1) | E08177 |
| lysC^{FBR}-E08178 | lysC A279T | JP 1994261766-A (Sequenz 2) | E08178 |
| lysC^{FBR}-E08179 | lysC A279V | JP 1994261766-A (Sequenz 3) | E08179 |
| lysC^{FBR}-E08180 | lysC S301F | JP 1994261766-A (Sequenz 4) | E08180 |
| lysC^{FBR}-E08181 | lysC T308I | JP 1994261766-A (Sequenz 5) | E08181 |
| lysC^{FBR}-E08182 | | JP 1994261766-A (Sequenz 6) | E08182 |
| lysC^{FBR}-E12770 | | JP 1997070291-A (Sequenz 13) | E12770 |
| lysC^{FBR}-E14514 | | JP 1997322774-A (Sequenz 9) | E14514 |
| lysC^{FBR}-E16352 | | JP 1998165180-A (Sequenz 3) | E16352 |
| lysC^{FBR}-E16745 | | JP 1998215883-A (Sequenz 3) | E16745 |
| lysC^{FBR}-E16746 | | JP 1998215883-A (Sequenz 4) | E16746 |
| lysC^{FBR}-I74588 | | US 5688671-A (Sequenz 1) | I74588 |
| lysC^{FBR}-I74589 | lysC A279T | US 5688671-A (Sequenz.2) | I74589 |
| lysC^{FBR}-I74590 | | US 5688671-A (Sequenz 7) | I74590 |
| lysC^{FBR}-I74591 | lysC A279T | US 5688671-A (Sequenz 8) | I74591 |
| lysC^{FBR}-I74592 | | US 5688671-A (Sequenz 9) | I74592 |
| lysC^{FBR}-I74593 | lysC A279T | US 5688671-A (Sequenz 10) | I74593 |
| lysC^{FBR}-I74594 | | US 5688671-A (Sequenz 11) | I74594 |
| lysC^{FBR}-I74595 | lysC A279T | US 5688671-A (Sequenz 12) | I74595 |
| lysC^{FBR}-I74596 | | US 5688671-A (Sequenz 13) | I74596 |
| lysC^{FBR}-I74597 | lysC A279T | US 5688671-A (Sequenz 14) | I74597 |
| lysC^{FBR}-X57226 | lysC S301Y | EP0387527 Kalinowski et al., Molecular and General Genetics 224:317-324 (1990) | X57226 |
| lysC^{FBR}-L16848 | lysC G345D | Follettie and Sinskey NCBI Nucleotide Database (1990) | L16848 |
| lysC^{FBR}-L27125 | lysC R320G lysC G345D | Jetten et al., Applied Microbiology Biotechnology 43:76-82 (1995) | L27125 |
| lysC^{FBR} | lysC T311I | WO0063388 (Sequenz 17) | |
| lysC^{FBR} | lysC S301F | US3732144 | |
| lysC^{FBR} | lysC S381F | EP0435132, | |
| lysC^{FBR} | lysC S317A | US5688671 (Sequenz 1) | |
| lysC^{FBR} | lysC T380I | WO 01/49854 | |

L-Lysin ausscheidende coryneforme Bakterien besitzen typischerweise einen oder mehrere der in Tabelle 1 aufgelisteten Aminosäureaustausche.

Bevorzugt werden folgende lysC^{FBR}-Allele: lysC A279T (Austausch von Alanin an Position 279 des kodierten Aspartatkinaseproteins gemäß SEQ ID NO: 20 gegen Threonin), lysC A279V (Austausch von Alanin an Position 279 des kodierten Aspartatkinaseproteins gemäß SEQ ID NO: 20 gegen Valin), lysC S301F (Austausch von Serin an Position 301 des kodierten Aspartatkinaseproteins gemäß SEQ ID NO: 20 gegen Phenylalanin), lysC T308I (Austausch von Threonin an Position 308 des kodierten Aspartatkinaseproteins gemäß SEQ ID NO: 20 gegen Isoleucin), lysC S301Y (Austausch von Serin an Position 308 des kodierten Aspartatkinaseproteins gemäß SEQ ID NO: 20 gegen Tyrosin), lysC G345D (Austausch von Glycin an Position 345 des kodierten Aspartatkinaseproteins gemäß SEQ ID NO: 20 gegen Asparaginsäure), lysC R320G (Austausch von Arginin an Position 320 des kodierten Aspartatkinaseproteins gemäß SEQ ID NO: 20 gegen Glycin), lysC T311I (Austausch von Threonin an Position 311 des kodierten Aspartatkinaseproteins gemäß SEQ ID NO: 20 gegen Isoleucin), lysC- S381F (Austausch von Serin an Position 381 des kodierten Aspartatkinaseproteins gemäß SEQ ID NO: 20 gegen Phenylalanin) und lysC S317A (Austausch von Serin an Position 317 des kodierten Aspartatkinaseproteins gemäß SEQ ID NO: 20 gegen Alanin).

Besonders bevorzugt werden das lysC^{FBR}-Allel lysC T311I (Austausch von Threonin an Position 311 des kodierte Aspartatkinaseproteins gemäß SEQ ID NO: 20 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 20 gegen Threonin) und S317A (Austausch von Serin an Position 317 des kodierten Aspartatkinaseproteins gemäß SEQ ID NO: 20 gegen Alanin).

Das lysC^{FBR}-Allel lysC T311I ist in dem bei der DSMZ unter der Nummer DSM16833 hinterlegten Stamm DM1797 enthalten. DM1797 ist eine Mutante von Corynebacterium glutamicum ATCC13032.

Ein lysC^{FBR}-Allel das den Aminosäureaustausch A279T enthält ist in dem bei der NRRL unter der Nummer NRRL B-11474 hinterlegten Mutante enthalten.

Nach der oben beschriebenen Art und Weise wurde, ausgehend von Stamm DM1797, eine als DM1825 bezeichnete Mutante isoliert, die ein opcA-Allel enthält, das für ein OpcA-Polypeptid kodiert, bei dem an Position 107 der Aminosäuresequenz L-Histidin, an Position 219 L-Asparagin, an Position 233 L-Serin und an Position 261 L-Histidin enthalten ist. Die Nukleotidsequenz der Kodierregion des opcA-Allels der Mutante DM1825 ist als SEQ ID NO:7 und die. Aminosäuresequenz des kodierten Polypeptids als SEQ ID NO:8 beziehungsweise 10 dargestellt. SEQ ID NO:7 enthält an Position 319 Cytosin anstelle Thymin, an Position 402 Thymin anstelle Cytosin, an Position 600 Cytosin anstelle Thymin, an Position 648 Cytosin anstelle Guanin, an Position. 657 .Cytosin anstelle Adenin, an Position 697 Thymin anstelle Cytosin und an Position 781 Cytosin anstelle Thymin. Die Formulierung "an Position 648 Cytosin anstelle Guanin" - und vergleichbare Formulierungen-bedeutet, dass die in der Wildtypsequenz der Kodierregion an Position 648 vorhandene Nukleobase Guanin (Siehe SEQ ID NO:1) durch Cytosin ersetzt worden ist (Siehe SEQ ID NO:7).

In der SEQ ID NO:9 sind die stromaufwärts (upstream) und stromabwärts (downstream) von SEQ ID NO:7 gelegenen Nukleotidsequenzen mit angegeben.

Darüberhinaus können L-Lysin ausscheidende coryneforme Bakterien verwendet werden, die eine abgeschwächte Homoserin-Dehydrogenase oder Homoserinkinase aufweisen oder andere Eigenschaften besitzen, wie sie aus dem Stand der Technik bekannt sind.

L-Tryptophan produzierende coryneforme Bakterien besitzen typischerweise eine "feed back" resistente beziehungsweise desensibilisierte Anthranilatsynthase. Unter "feed back" resistenter Anthranilatsynthase versteht man Anthranilatsynthasen, die im Vergleich zur Wildform eine geringere Empfindlichkeit gegenüber der Hemmung (5 bis 10%, 10% bis 15% oder 10% bis 20%)durch Tryptophan oder 5-Fluorotryptophan (Matsui et al., Journal of Bacteriology 169 (11): 5330 - 5332 (1987)) oder ähnliche Analoga aufweisen.. Die für diese desensibilisierten Anthranilatsynthasen kodierenden Gene beziehungsweise Allele werden auch als trpE^{FBR} -Allele bezeichnet. Beispiele für derartige Mutanten beziehungsweise Allele sind beispielsweise in der US 6180373 und EP0338474 beschrieben.

Die erhaltenen Mutanten zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Gegenstand der Erfindung ist ebenfalls ein isoliertes Polynukleotid, das für ein OpcA-Polypeptid der Glucose-6-Phosphat-Dehydrogenase kodiert, dadurch gekennzeichnet, dass die Aminosäuresequenz SEQ ID NO:6 des Polypeptids folgende Aminosäuren aufweist:
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin.

Das erfindungsgemäße Polynukleotid kann aus einer erfindungsgemäßen Mutante isoliert werden.

Weiterhin können für die Mutagenese des opcA-Gens in-vitro Methoden eingesetzt werden. Bei der Verwendung von in-vitro Methoden werden isolierte Polynukleotide, welche ein opcA-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA beziehungsweise chromosomale DNA oder auch um Amplifikate des opcA-Gens handeln, die mit Hilfe der Polymerasekettenreaktion (PCR) hergestellt wurden. Derartige Amplifikate werden auch als PCR-Produkte bezeichnet; der Begriff Nukleinsäurefragment ist ebenfalls gebräuchlich. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich, das zu mutagenisierende opcA-Gen zunächst in einen Vektor, beispielsweise in einen Bakteriophagen oder in ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von.Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Gegenstand der Erfindung ist weiterhin ein isoliertes Polynukleotid, das für ein OpcA-Polypeptid kodiert, welches die Aminosäuresequenz des SEQ ID NO:6 mit einer Länge von 319 Aminoäuren umfasst und folgende Aminosäuren enthält:
a) an Position 107 L-Histidin,
b) an Position 219 L-Asparagin,
c) an Position 233 L-Serin, und
d) an Position 261 L-Histidin.

Gegenstand der Erfindung ist weiterhin ein isoliertes Polynukleotid, dass für ein OpcA-Polypeptid kodiert, das eine Aminosäuresequenz entsprechend Position 98 bis 270 von SEQ ID NO:6 oder Position 83 bis 285 von SEQ ID NO:6 oder Position 63 bis 305 von SEQ ID NO:6 oder Position 38 bis 315 von SEQ ID NO:6 oder Position 8 bis 315 von SEQ ID NO:6 oder Postition 2 bis 315 von SEQ ID NO:6 oder Position 2 bis 317 von SEQ ID NO:6 oder Position, 2 bis 319 von SEQ ID NO:6 enthält. Ganz besonders bevorzugt umfasst die Länge des kodierten Polypeptids die 319 Aminosäuren der SEQ ID NO:6.

Gegenstand der Erfindung ist weiterhin ein isoliertes Polynukleotid, dass für ein OpcA-Polypeptid kodiert, das dadurch gekennzeichnet ist, dass die Aminosäuresequenz des Polypeptids aus der SEQ ID NO:6 besteht, in der sich
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin befinden
und das eine Nukleotidsequenz umfasst, welche mit der Nukleotidsequenz eines Polynukleotids identisch ist, das durch eine Polymerasekettenreaktion (PCR) unter Verwerdung des Primerpaars erhältlich ist, deren Nukleotidsequenzen jeweils mindestens 15 aufeinanderfolgende Nukleotide umfassen, die aus der Nukleotidsequenz zwischen Position 1 und 334 von SEQ ID NO:3 und aus der komplementären Nukleotidsequenz zwischen Position 1600 und 1292 von SEQ ID NO:3 ausgewählt werden.. Ein Beispiel für geeignetes Primerpaar ist in SEQ ID NO:11 und SEQ ID NO:12 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Gegenstand der Erfindung ist weiterhin ein isoliertes Polynukleotid, das mit der zu SEQ ID NO:5 oder 7 komplementären Nukleotidsequenz unter stringenten Bedingungen hybridisiert und für ein OpcA-Polypeptid kodiert, dessen Aminosäuresequenz aus der SEQ ID NO:6 besteht, in der sich
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin befinden.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of, Systematic Bacteriology 41: 255-260 (1991)). Die Hybridisierung findet unter stringenten Bedingungen statt, das -heißt, es werden nur Hybride gebildet, bei denen die Sonde d.h. ein Polynukleotid umfassend die zu SEQ ID NO:5, 7 oder 9 komplementäre Nukleotidsequenz und die Zielsequenz, d. h. die mit der Sonde behandelten beziehungsweise identifizierten Polynukleotide, mindestens 90% identisch sind. Es ist bekannt, dass die Stringenz der Hybridisierung einschließlich der Waschschritte durch Variieren der Puffer.zusammensetzung, der Temperatur und der Salzkonzentration beeinflusst bzw. bestimmt wird. Die. Hybridisierungsreaktion wird im Allgemeinen bei..relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 90% Identität zur Nukleotidequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca.. 64°C - 68°C, ca. 66°C - 68°C eingestellt wird. Temperaturbereiche von ca.. 64°.C - 68°C oder ca. 66°C - 68°C werden bevorzugt.. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 90% oder mindestens 91%, bevorzugt mindestens 92% oder mindestens 93% oder mindestens 94% oder mindestens 95% oder mindestens 96% und ganz besonders bevorzugt mindestens 97% oder mindestens 98% oder mindestens 99% Identität zur Sequenz beziehungsweise komplementären Sequenz der eingesetzten Sonde besitzen und für ein OpcA-Polypeptid kodieren, welches den erfindungsgemäßen Aminosäureaustausch enthält. Die Nukleotidsequenz des auf diese Weise erhaltenen Polynukleotids wird mit bekannten Methoden bestimmt. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für OpcA-Polypeptide, die mindestens 90% bevorzugt mindestens 92% oder mindestens 94% oder mindestens 96%, und ganz besonders bevorzugt mindestens 97% oder mindestens 98% oder mindestens 99% identisch sind mit der Aminosäuresequenz von SEQ ID NO:6 oder SEQ ID NO:2 und die vier erfindungsgemäßen Aminosäureaustausche enthalten.. Gegenstand der Erfindung ist weiterhin ein isoliertes Polynukleotid, das für das Polypeptid der Glucose-6-Phosphat-Dehydrogenase kodiert, wobei die Amino.säuresequenz des Polypeptids, ausgewählt ist aus der Gruppe, bestehend aus
a) SEQ ID NO: 6, in der sich an Position 107 der Aminosäuresequenz L-Histidin
   an Position 219 L-Asparagin
   an Position 233 L-Serin und
   an Position 261 L-Histidin befinden
b) SEQ ID NO: 6, und sich über die genannten Mutationen hinaus maximal 5 konservative Aminosäureaustausche,
c) SEQ ID NO: 6, in der sich über genannten Mutationen hinaus maximal 5 Insertionen oder Deletionen finden.

Gegenstand der Erfindung ist weiterhin ein isoliertes Polynukleotid, das für ein OpcA-Polypeptid kodiert, das dadurch gekennzeichnet ist, dass die Aminosäuresequenz des Polypeptids aus der SEQ ID NO:6 besteht, in der sich
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäüresequenz L-Histindin befinden,
und das eine Nukleötidsequenz umfasst, die außerdem zu mindestens 90%, bevorzugt mindestens 92% oder mindestens 94% mindestens 96%, und ganz besonders bevorzugt mindestens 97% oder mindestens 98% oder mindestens 99% identisch ist mit der Nukleotidsequenz von SEQ ID NO:5.

Ein Beispiel für ein Polynukleotid, das für ein erfindungsgemäßes OpcA-Polypeptid kodiert, und eine Nukleotidsequenz besitzt, die mindestens 99% identisch ist mit der von SEQ ID NO:5, ist in SEQ ID NO:7 gezeigt. Die Nukleotidsequenz dieses opcA-Allels besitzt zusätzlich zu den Nukleotidaustauschen Thymin gegen Cytosin an Position 319, Adenin gegen Cytosin an Position 657, Cytosin gegen Thymin an Position 697 und Thymin gegen Cytosin an Position 781 (siehe SEQ ID NO:5) die Nukleotidaustausche Cytosin gegen Thymin an Position 402, Thymin gegen Cytosin an Position 600, und Guanin gegen Cytosin an Position 648 (siehe SEQ ID NO:7). Die Formulierung "Guanin gegen Cytosin an der Position 648" - und vergleichbare Formulierungen - bedeutet, dass die in der-Wildtypsequenz der Kodierregion an der Position 648 vorhandene Nukleobase Guanin (Siehe SEQ ID NO:1) durch Cytosin ersetzt worden ist (Siehe SEQ ID NO:7).

Gegenstand der Erfindung ist weiterhin ein isoliertes Polynukleotid, dass für, ein opcA-Polypeptid kodiert, welches die Aminosäuresequenz von SEQ ID NO:6 einschließlich einer Verlängerung am N- oder C-Terminus,um ., mindestens eine (1) Aminosäure umfasst. Diese Verlängerung beträgt nicht mehr als 50, 40, 30, 20, 10, 5, 3 oder 2 Aminosäuren beziehungsweise Aminosäurereste.

Gegenstand der Erfindung ist weiterhin ein isoliertes Polynukleotid, das die Nukleotidsequenz gemäß SEQ ID NO: 5 oder 7 umfasst.

Gegenstand der Erfindung ist schließlich ein isoliertes. Polynukleotid enthaltend das opcA-Allel der Mutante DM1825.

Gegenstand der Erfindung ist außerdem ein isoliertes Polynukleotid, das einen Teil der Kodierregion eines erfindungsgemäßen opcA-Allels umfasst, wobei das isolierte Polynukleotid in jedem Fall den Teil der Kodierregion umfasst, der die vier erfindungsgemäßen Amihosäureaustausche enthält. Der Begriff "Teil der Kodieregion" bedeutet, dass entweder das Startkodon (typischerweise ATG oder GTG), oder das letzte kodierende Kodon (im vorliegenden Fall GTC entsprechend Position 955 - 957 von SEQ ID NO:5), oder beide in dem isolierten Polynukleotid fehlen.

Bevorzugt werden Nukleinsäuremoleküle, die für mindestens eine Aminosäuresequenz entsprechend Position 98 bis 270 von SEQ ID NO:6, oder mindestens entsprechend Position 83 bis 285 von SEQ ID NO:6, oder mindestens entsprechend Position 63 bis 305 von SEQ ID NO:6, oder mindestens entsprechend Position 38 bis 315 von SEQ ID NO:6, oder mindestens entsprechend Position 8 bis 315 von SEQ ID NO:6 kodieren.

Das Leseraster kodierend für die Aminosäuresequenz entsprechend Position 98 bis 270 von SEQ ID NO:6 ist ebenfalls als SEQ ID NO:15 dargestellt. SEQ ID NO:16 zeigt die von diesem Leseraster kodierte Aminosäuresequenz. Die Position 10 in SEQ ID-NO:16 entspricht der Position 107 von SEQ ID NO:2, 4, 6, 8 oder 10. Die Position 122 in SEQ ID NO:16 entspricht der Position 219 von SEQ ID NO:2, 4, 6, 8 oder 10. Die Position 136 in SEQ ID NO:16 entspricht der Position 233 von SEQ ID NO:2, 4, 6, 8 oder 10. Die Position 164 in SEQ ID NO:16 entspricht der Position 261 von SEQ ID NO:2, 4, 6, 8 oder 10.

Ganz besonders bevorzugt werden Nukleinsäuremoleküle, die mindestens eine Nukleotidsequenz entsprechend Position 292 bis 810 von SEQ ID NO:5 oder 7, oder mindestens eine Nukleotidsequenz entsprechend Position 247 bis 855 von SEQ ID NO:5 oder 7, oder mindestens eine Nukleotidsequenz entsprechende Position187 bis 915 von SEQ ID NO:5 oder 7, oder mindestens eine Nukleotidsequenz entsprechend Position 112 bis 948 von SEQ ID NO:5 oder 7, oder mindestens eine Nukleotidsequenz entsprechend Position 22 bis 948 von SEQ ID NO:5 oder 7 umfassen.

Das Leseraster entsprechend Position 292 bis 810 von SEQ ID, NO:5 ist als SEQ ID NO:15 dargestellt. Die dazugehörige kodierte Aminosäuresequenz ist als SEQ ID NO:16 dargestellt. Das Leseraster entsprechend Position 292, bis 810 von SEQ ID NO:7 ist als SEQ ID NO:17 dargestellt. Die dazugehörige kodierte Aminosäuresequenz ist als SEQ ID NO:18 dargestellt.

Die erfindungsgemäßen Leseraster können darüberhinaus eine oder mehrere Mutationen enthalten, die zu maximal fünf konservativen Aminosäureaustauschen führen.

Bevorzugt führen die Mutationen zu maximal 4%, zu maximal 2% oder zu maximal 1% konservativen Aminosäureaustauschen. Weiterhin können die erfindungsgemäßen Leseraster eine oder mehrere stille Mutationen enthalten. Bevorzugt enthalten die erfindungsgemäßen Leseraster höchstens 4% und besonders bevorzugt höchstens 2% bis höchstens 1% stille Mutationen.

Die erfindungsgemäßen, isolierten Polynukleotide können dazu verwendet werden, um rekombinante Stämme von Mikroorganismen herzustellen, die im Vergleich zum Ausgangs- beziehungsweise Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums überführt und die Mutation durch wenigstens zwei Rekombinationsereignisse beziehungsweise "cross over"-Ereignisse in das Chromosom des gewünschten Stammes inkorporiert beziehungsweise die im betreffenden Stamm vorhandene Sequenz eines Gens gegen die _mutierte Sequenz ausgetauscht.

Von Schwarzer und Pühler (Bio/Technology 9, 84-87 (1991) wurde diese Methode verwendet um ein lysA-Allel, welches eine Deletion trug, in das Chromosom von C. glutami.cum anstelle des Wildtypgens einzubauen. In gleicher Weise wurde ein lysA-Allel welches eine Insertion trug.in das: Chromosom von C- glutamicum anstelle des Wildtypgens eingebaut. Von Schäfer et al. (Gene 145, 69-73 (1994)) wurde diese Methode eingesetzt um eine Deletion in das homthrB Operon von C. glutamicum einzubauen. Von Nakagawa et al. (EP 1108790) und Ohnishi et al. (Applied Microbiology and Biotechnology 58(2), 217-223 (2002)) wurde diese Methode eingesetzt um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutation in das als Glucose-6-phoshat-Dehydrogenase Gen bezeichnete zwf-Gen von C.. glutamicum Stämmen einzubauen.

Für ein erfindungsgemäßes Verfahren kann ein erfindungsgemäßes Polynukleotid verwendet werden, welches. die gesamte Kodierregion umfasst, wie beispielsweise in SEQ ID NO:5 oder 7 gezeigt, oder welches einen Teil der Kodierregion umfasst, wie beispielsweise die Nukleotidsequenz, die für mindestens die Aminosäuresequenz entsprechend Position 98 bis 270 von SEQ ID NO:6 beziehungsweise 8 kodiert und die als SEQ ID NO:15 und 17 dargestellt sind. Der Teil der Kodierregion entsprechend mindestens SEQ ID NO:15 oder 17 hat eine Länge von ≥519 Nukleobasen. Bevorzugt werden solche Teile der Kodierregion, deren Länge ≥ 747 Nukleobasen beträgt, wie beispielsweise Nukleinsäuremoleküle, die für mindestens, eine Aminosäuresequenz entsprechend Position von 63 bis 305 von SEQ ID NO: 6 beziehungsweise 8 kodieren. Ganz besonders bevorzugt werden solche Teile der Kodierregion, deren Länge ≥ 834 Nukleobasen beträgt, wie beispielsweise Nukleinsäuremoleküle, die für mindestens eine Aminosäuresequenz entsprechend Position von 38 bis 315 von SEQ ID NO:6 beziehungsweise 8 kodierten.

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia, bevorzugt der Spezies Escherichia coli, verwendet.

Beispiele für,derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli, aber nicht in coryneformen Bakterien replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise .Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird- Weiterhin wurde von Nakamura et al. (US-A-6,303,383) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium überführt. Gegebenenfalls kann die Überführung der DNA auch durch Partikelbeschuss, erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Ein weiterer Gegenstand der Beschreibung ist dementsprechend ein Verfahren zur Herstellung eines coryneformen Bakteriums, bei dem man
a) ein erfindungsgemäßes Polynukleotid in ein coryneformes Bakterium überführt,
b) das im Chromosom des coryneformen Bakteriums vorhandene opcA-Gen, das für eine Aminosäuresequenz mit L-Tyrosin an Position 107, L-Lysin an Position 219, L-Prolin an Position 233 und L-Tyrosin an Position 261 kodiert, gegen das Polynukleotid aus a) austauscht,
c) das gemäß Schritt a) und b) erhaltene coryneforme Bakterium vermehrt.

Auf diese Weise erhält man ein rekombinantes coryneformes. Bakterium, welches anstelle des Wildtyp opcA-Gens ein (1) erfindungsgemäßes opcA-Allel enthält.

Ein weiteres Verfahren zur Herstellung eines Mikroorganismus besteht darin, dass man
a) ein erfindungsgemäßes Polynukleotid, das für ein opcA-Polypeptid kodiert, in einen Mikrorganismus überführt,
b) das Polynukleotid in dem Mikroorganismus repliziert, und
c) den gemäß Schritt a) und b) erhaltenen Mikroorganismus vermehrt.

Auf diese Weise erhält man einen rekombinanten Mikroorganismus, welcher mindestens eine (1) Kopie oder mehrere Kopien eines erfindungsgemäßen Polynukleotids enthält, das für eine opcA-Polypeptid kodiert, das dadurch gekennzeichnet ist, dass die Aminosäuresequenz des polypeptids eine oder mehrere der Aminosäuren aufweist, ausgewählt aus der Gruppe:
a) an Position 107 der Aminosäuresequenz L-Histidin,
b) an Position 219 der Aminosäuresequenz L-Asparagin,
c) an Position 233 der Aminosäuresequenz L-Serin, und
d) an Position 261 der Aminosäuresequenz L-Histidin.

Weitere Gegenstände der Erfindung sind dementsprechend Wirte beziehungsweise Wirtszellen, bevorzugt Mikroorganismen, besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet.. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Die erfindungsgemäßen, isolierten Polynukleotide können ebenfalls zur Erzielung einer Überexpression der von ihnen kodierten Polypeptide verwendet werden.

Unter Überexpression versteht man allgemein eine Erhöhung der.intrazellulären Konzentration oder Aktivität einer Ribonukleinsäure, eines Proteins oder eines Enzyms. Im Falle der vorliegenden Erfindung werden opcA-Allele beziehungsweise Polynukleotide überexprimiert, die für die erfindungsgemäß OpcA-Polypeptide kodieren.

Es ist bekannt, dass durch wirtseigene Enzyme - sogenannte Aminopeptidasen - N-terminale Aminosäuren, insbesondere das N-terminale Methionin, vom gebildeten Polypeptid abgespalten werden können.

Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al.. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132 (1994)) für das hom-thrB-Operon beschrieben ist, wird ein in C.. glutamicum nicht-replikatives Plasmid, welche das interessierende Gen enthält, in ein coryneformes Bakterium überführt. Nach homologer Rekombination mittels eines "cross over"-Ereignisses enthält der resultierende Stamm mindestens zwei Kopien des betreffenden Gens beziehungsweise Allels..

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eines lysC-Allels, das für eine L-Lysin insensitive Aspartatkinase kodiert, in das gluB-Gen von C. glutamicum eingebaut..

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines lysC^{FBR}-Allels am natürlichen lysC-Genort erzielt..

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. Weiterhin können die hinlänglich bekannten von Amann et al. (Gene 69(2), 301-315 (1988)) und Amann und Brosius (Gene 40(2-3); 183-190 (1985)) beschriebenen Promotoren T3, T7, SP6, M13, lac, tac und trc verwendet werden. Ein derartiger Promotor kann beispielsweise stromaufwärts der Kodierregion eines erfindungsgemäßen opcA-Allels, typischerweise im Abstand von ungefähr 1 - 500 oder 1 -334 Nukleotiden vom Startkodon, eines rekombinanten coryneformen Bakteriums eingefügt werden. Ein derartiger Promotor kann naturgemäß ebenfalls stromaufwärts des opcA-Allels einer erfindungsgemäßen Mutante eingefügt werden. Es ist weiterhin möglich ein erfindungsgemäßes isoliertes Polynukleotid, das für eine erfindungsgemäße Variante des OpcA-Polypeptides kodiert, mit einem Promotor zu verknüpfen und die erhaltene Expressionseinheit in ein extrachromosomal replizierendes Plasmid oder in das Chromosom eines coryneformen Bakteriums einzubauen.

Darüberhinaus kann die Promotor- und Regulationsregion oder die Ribosomenbindungsstelle, die sich stromaufwärts des Strukturgens befindet, mutiert werden. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Külturführung erreicht werden.

Durch die Maßnahmen der Überexpression wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus beziehungsweise Elternstammes erhöht. Unter einem Ausgangs-Mikroorganismus oder Elternstamm versteht man einen Mikroorganismus, an dem die Massnahmen der Erfindung durchgeführt werden.

Die Konzentration des Proteins kann über 1- und 2- dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden.

Gegenstand der Erfindung sind dementsprechend Verfahren zur Überexpression der erfindungsgemäßen OpcA-Polypeptide. Ein erfindungsgemäßes Verfahren zur Überexpression besteht unter anderem darin, dass man die Kopienzahl eines erfindungsgemäßen Polynukleotids, das für eine erfindungsgemäße OpcA-Polypeptid-Variante kodiert, um mindestens eine (1) oder um mehrere Kopien erhöht. Ein weiteres erfindungsgemässes Verfahren besteht darin, dass man einen Promotor funktionell mit dem Polynukleotid verknüpft.

Gegenstand der Erfindung sind weiterhin Mikroorganismen, die eine erhöhte Konzentration eines erfindungsgemäßen OpcA-Polypeptides in ihrem Zellinneren aufweisen. Gegebenenfalls besitzen diese Mikrorganismen eine erhöhte Aktivität der Glucose-6-Phosphat-Dehydrogenase in ihrem Zellinneren.

Zusätzlich kann es für die verbesserte Produktion von L-Aminosäuren vorteilhaft sein, in den erfindungsgemäßen Mutanten oder rekombinanten Stämme eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine überzuexprimieren. Die Verwendung endogener Gene wird im allgemeinen bevorzugt.

Unter ,,endogenen Genen" oder "endogenen Nukleotidsequenzen" versteht man die in der Population einer Art vorhandenen Gene beziehungsweise Nukleotidsequenzen oder Allele.

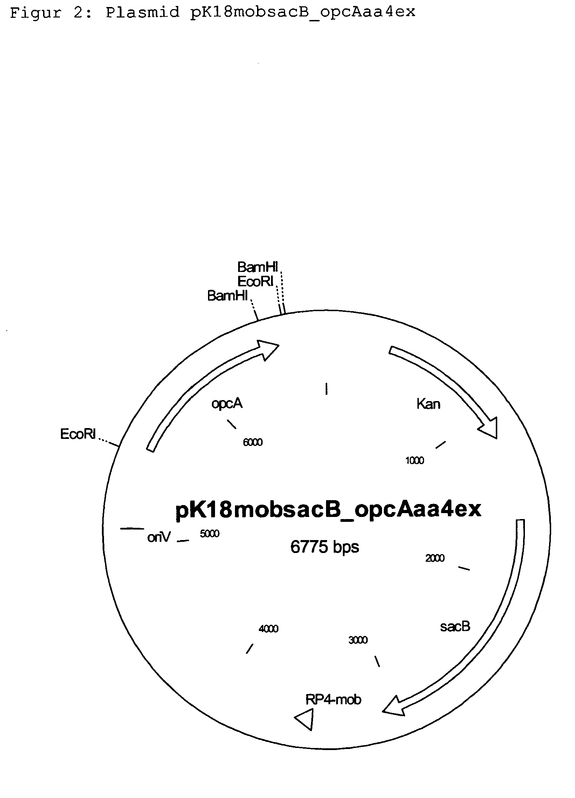

So kann für die Herstellung von L-Lysin eines oder mehrere der Gene, ausgewählt aus der Gruppe
- ein für ein Dihydrodipicolinat-Synthase kodierendes Gen dapA, wie beispielsweise das in der EP 0 197 335 beschriebene dapA-Gen des Wildtyps von Corynebacterium glutamicum,
- ein für eine Glucose-6-Phosphat Dehydrogenase kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist,
- ein für eine Pyruvat-Carboxylase kodierendes Gen pyc, wie beispielsweise das in der DE-A-198 31 609 und EP 1108790 beschriebene pyc-Gen des Wildtyps von Corynebacterium glutamicum,
- das für das in der EP 1 108 790 beschriebene pyc-Allel von Corynebacterium glutamicum, das für ein Protein kodiert, bei dem L-Prolin an Position 458 der Aminosäuresequenz durch L-Serin ersetzt ist,
- die in der WO 02/31158 beschriebene pyc-Allele von Corynebacterium glutamicum, die für Proteine kodieren, welche gemäß Aspruch 1 einen oder mehrere der Aminosäureaustausche ausgewählt aus der Gruppe L-Glutaminsäure an Position 153 ersetzt durch L-Asparaginsäure, L-Alanin an' Position 182 ersetzt durch L-Serin, L-Alanin an Position 206 ersetzt durch L-Serin, L-Histidin an Position 227 ersetzt durch L-Arginin, L-Alanin an Position 452 ersetzt durch Glycin und L-Asparaginsäure an Position 1120 ersetzt durch L-Glutaminsäure tragen (Figur 2A von WO 02/31158 gibt zwei verschiedene Startpositionen für die Pyruvat-Carboxylase an, die sich um eine Länge entsprechend 17 Aminosäuren unterscheiden. Dementsprechend entspricht die Position 153 nach Anspruch 1 von WO 02/31158 der Position 170 von Fig.2A von WO 02/31158, die Position 182 nach Anspruch 1 der Position 199 von Fig.2A, die Position 206 nach Anspruch 1 der Position 223 von Fig. 2A, die Position 227 nach Anspruch 1 der Position 244 von Fig.2A, die Position 452 nach Anspruch 1 der Position 469 von Fig. 2A, die Position 1120 nach Anspruch 1 der Position 1137 von Fig. 2B. In Figur 2A von WO 02/31158 ist weiterhin ein Aminosäureaustausch A (Alanin) gegen G (Glycin) an Position 472 angegeben. Die Position 472 des Proteins mit der N terminalen Sequenz MTA entspricht der Position 455 des Proteins mit der N-terminalen Sequenz MST gemäß Fig. 2A. In Fig. 2B von WO 02/31158 ist weiterhin ein Aminosäureaustausch D (Asparaginsäure) gegen E (Glutaminsäure) an Position 1133 des Proteins mit dem N-Terminus MTA angegeben.),
- ein für eine Aspartatkinase kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 .(Siehe Zugangsnummer AX063743) beschriebene von lysC-Gen des Wildtyps von Corynebacterium glutamicum,
- ein für eine feed-back resistente Aspartatkinase Variante kodierendes lysC^{FBR} Allel, insbesondere entsprechend Tabelle 1,
- ein für ein Lysin-Export-Protein kodierendes Gen lysE, wie beispielsweise das in der DE-A-195 48 222 beschriebene lysE-Gen von des Wildtyps Corynebacterium glutamicum,
- das für das Zwa1-Protein kodierende Gen zwa1 des Wildtyps von Corynebacterium glutamicum (US 6,632,644)
überexprimiert werden.

Weiterhin kann es für die Produktion von L-Lysin vorteilhaft sein, neben der Verwendung der erfindungsgemäßen Allele des opcA-Gens gleichzeitig eines oder mehrere der endogenen Gene, ausgewählt aus der Gruppe
- ein für die Glucose-6-Phosphat-Isomerase kodierendes Gen pgi, wie beispielsweise das in der US 6,586,214 und US 6,465,238 beschriebene pgi-Gen von Corynebacterium glutamicum,
- ein für die Homoserin-Dehydrogenase kodierendes Gen hom, wie beispielsweise das in der EP-A-0131171 beschriebene hom-Gen von Corynebacterium glutamicum,
- ein für die Homoserin-Kinase kodierendes Gen thrB, wie beispielsweise das von Peoples et al. (Molecular Microbiology 2 (1988): 63 - 72)) beschriebene thrB-Gen von Corynebacterium glutamicum und
- ein für die Phosphofruktokinase kodierendes Gen pfkB, wie beispielsweise das in der WO 01/00844 (Sequenz Nr. 57) beschriebene pfkB-Gen von Corynebacterium glutamicum,
abzuschwächen oder auszuschalten.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw.. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stopp-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stopp-Kodon im Kodierbereich, so führt dies ebenfalls zu einem vorzeitigen Abbruch der Translation.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6.. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Die durch die Massnahmen der Erfindung erhaltenen isolierten coryneformen Bakterien zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Unter isolierten Bakterien sind die erfindungsgemäßen, isolierten beziehungsweise erzeugten Mutanten und rekombinanten Bakterien, insbesonders coryneformer Bakterien, zu verstehen, welche ein opcA-Allel enthalten, das für ein erfindungsgemäßes OpcA-polypeptid kodiert.

Die Leistung der isolierten Bakterien bzw. des Fermentätionsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgarigstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch. (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D..C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben-oder Zuckerröhrherstellüng, Stärke, Stärkehydrolysat und Cellulose, Öle und Fette, wie zum Bespiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werde.

Als Stickstoffquelle können organische Stickstoff-haltige. Verbindungen wie Peptone; Hefeextrakt, Fleischextrakt, Malzextrakt, Maisquellwasser, Sojabohnenmehl und Harnstoff; oder anorganische Verbindungen wie Ammoniumsulfat, Ammoniumchlorid; Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt- Der pH wird im Allgemeinen auf einen Wert von 6, 0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung.der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die.Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie ' mit anschließender Ninhydrin-Derivatisierung erfolgen, oder sie kann durch reversed phase HPLC erfolgen, so wie bei Lindroth et al. (Analytical. Chemistry (1979) 51: 1167-1174) beschrieben.

Gegenstand der Erfindung ist dementsprechend ein Verfahren zur Herstellung einer L-Aminosäure bei dem man
a) ein isoliertes coryneformes Bakterium in einem geeignetem. Medium fermentiert, wobei das Bakterium ein für ein erfindungsgemäßes OpcA-Polypeptid kodierendes Gen enthält, und
b) die L-Aminasäure in der Fermentationsbrühe oder in den Zellen des isolierten coryneformen Bakteriums anreichert.

Die auf diese Weise hergestellte Fermentationsbrühe wird anschließend gesammelt und vorzugsweise zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medium enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im wesentlichen vier verschiedene Produktformen bekannt.

Eine Gruppe L-Lysin haltiger Produkte umfasst konzentrierte, wässrige, alkalische Lösungen von aufgereinigtem L-Lysin (EP-B-053-4865). Eine weitere Gruppe, wie beispielsweise in der US 6,340,486 und US 6,465,025 beschrieben., umfasst wässrige, saure, Biomasse-haltige Konzentrate von L-Lysin-haltigen Fermentationsbrühen. Die bekannteste Gruppe fester Produkte umfasst pulverförmige oder kristallin Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0%-100%.

Entsprechend'der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenfalls unter Verwendung von Aktivkohle und Methoden der Kristallisierung angewendet. Im Falle des Lysin erhält man auf diese Weise die entsprechende Base oder ein entsprechendes Salz wie beispielsweise das Monohydrochlorid (Lys-HCl) oder das Zysinsulfat (Lys₂-H₂SO₄).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellte Die mineralischen Bestandteile (anorganischen Salze) werden nach Konzentrierung und Abkühlung durch Kristallisation aus der Brühe abgetrennt und entweder als Dünger verwendet oder verworfen.

Bei Verfähren zur Herstellung von Lysin unter Verwendung der.erfindungsgemäßen Bakterien werden solche Verfahren bevorzugt, bei denen Produkte erhalten werden, die Bestandteile der Fermentationsbrühe enthalten. Diese werden insbesondere als Tierfuttermittelädditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

Die chemischen Bestandteile der Biomasse sind unter anderem die Zellhülle, beispielsweise das Peptidoglykan und das Arabinogalaktan, das Protein bzw.. Polypeptid, beispielsweise das Glucose-6-Phosphat-Dehydrogenase Polypeptid, Lipide und Phospholipide und Nukleinsäuren (DNA und RNA), beispielsweise Polynukleotide enthaltend die erfindungsgemäße Mutation. Als Folge der Massnahmen der Inaktivierung und/oder der weitereren Verfahrensschritte (beispielsweise Ansäuerung,Sprühtrocknung, Granulation etc.) liegen Nukleinsäuren typischerweise als Fragmente mit eine Länge von unter anderem ≥ 40 - 60 bp, >60 - 80 bp, >80 - 100 bp, >100 - 200 bp, >200 - 300 bp, >300 - 400 bp, >400 - 500 bp, >500 - 750 bp, >750 - 1000 bp, >1000 -1250 bp, >1250 - 1500 bp, >1500 - 1750 bp, >1750 - 2000 bp, >2000-2500 bp, >2500 - 3000 bp, >3000 - 4000 bp, >4000 - 5000 bp vor.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern (US 6,340,486 oder US 6,465,025). Schließlich kann die Brühe auch durch Zusatz von Natriumbisulfit (NaHSO₃ GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt.. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeinte

Die Korngrößeribestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmarin, Wissenschaftliche Verlagsgesellschaft stuttgart (1996) oder im Lehrbuch "Introduction to Particle Technology" von M. Rhodes, Verlag Wiley & Sons (1998) beschrieben.

Das rieselfähige, feinteilige Pulver kann wiederum, durch geeignete'Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Ein weiterer Gegenstand der Erfindung ist dementsprechend ein Verfahren zur Herstellung eines L-Aminosäure, bevorzugt L-Lysin oder L-Tryptophan, haltigen Produktes, bevorzugt Tierfuttermittel-Additivs, aus Fermentationsbrühen, gekennzeichnet durch die Schritte
a) Kultivierung und Fermentation eines L-Aminosäure ausscheidenden coryneformen Bakteriums, welches mindestens ein opcA-Allel enthält, das für ein erfindungsgemäßes OpcA-Polypeptid kodiert, in einem Fermentationsmedium,
b) Entfernung der während der Fermentation gebildeten Biomasse in einer Menge von 0 bis 100 Gew.-%, und
c) Trocknung der gemäß a) und/oder b) erhaltenen Fermentationsbrühe, um das Produkt in der gewünschten Pulver- oder Granulatform zu erhalten
wobei gegebenenfalls vor Schritt b) oder c) eine Säure ausgewählt aus der Gruppe Schwefelsäure, Phosphorsäure oder Salzsäure hinzugefügt wird.

Vorzugsweise wird im Anschluss an Schritt a) oder b) Wasser aus der L-Aminosäure haltigen Fermentationsbrühe entfernt (Aufkonzentration).

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken' Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschriebene, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Im Falle des Lysin wird bei der Herstellung Lysin-haltiger Produkte das Verhältnis der Ionen bevorzugt so eingestellt, dass das Ionenverhältnis entsprechend nachstehender Formel

2x[SO₄²⁻]+[Cl⁻]-[NH₄⁺]-[Na⁺]-[K⁺]-2x[Mg⁺]-2x[Ca²⁺]/[L-Lys]

0,68 bis 0,95, bevorzugt 0,68 bis 0,90 ergibt so wie von Kushiki et al. in der US 20030152633 beschrieben.

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

Eine Mutante von Corynebacterium glutamicum mit der Bezeichnung DM1797, die den Aminosäureaustausch lysC T311I in der Aspartatkinase enthält, wurde am 28. Oktober 2004 bei der Deutschen Sammlung für Mikroorganismen und Zellkulturen (DSMZ, Braunschweig, Deutschland) als DSM 16833 hinterlegt.

Die erfindungsgemäße Mutante Corynebacterium glutamicum DM1825, die L-Histidin an Position 107, L-Lysin an Position 219, L-Prolin an Position 233 und L-Tyrosin an Position 261 der Aminosäuresequenz des OpcA-Polypeptids enthält, wurde am 05. April 2005 bei der Deutschen Sammlung für Mikroorganismen und Zellkulturen (DSMZ, Braunschweig, Deutschland) als DSM 17223 hinterlegt.

### Beispiel 1

### Mutagenese des L-Lysin produzierenden Stammes DM1797

Der Corynebacterium glutamicum Stamm DM1797 wurde als Ausgangsstamm für die Mutagenese mit N-Methyl-N'-Nitro-N-Nitrosoguanidin (MNNG) eingesetzt. Der Stamm DM1797 ist eine Aminoethylcystein-resistente Mutante von Corynebacterium glutamicum ATCC13032 und unter der Bezeichnung DSM16833 bei der Deutschen Sammlung für Mikroorganismen und Zellkulturen (DSMZ, Braunschweig, Deutschland) hinterlegt.

Der Stamm DM1797 wurde in 10 ml LB-Bouillon (Merck, Darmstadt, Deutschland), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen.. Die Zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 2500 Mutanten isoliert.

### Beispiel 2

### Leistungstest der Mutanten des Stammes DM1797

Die in Beispiel 1 erhaltenen Mutanten wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

| Medium MM | |
|---|---|
| CSL | g/l |
| MOPS | 20 g/l |
| Glucose (getrennt autoklaviert)' | 50 g/l |

| Salze: | |
|---|---|
| (NH₄)₂SO₄) | 25 g/l |
| KH₂PO₄ | 0,1 g/l |
| MgSO₄ * 7 H₂O | 1, 0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1825 bezeichnet.

**Tabelle 1**

| Stamm | OD (660) | Lysin-HCl (g/l) |
|---|---|---|
| DM1797 | 12,3 | 3,5 |
| DM1825 | 12,2 | 3,7 |

### Beispiel 3

### Sequenzierung des opcA-Gens der Mutante DM1825

Aus dem Klon DM1825 wurde mit der Methode von Eikmanns et al. (Microbiology 140: 1817 - 1828 (1994)) chromosomale DNA isoliert. Mit Hilfe der.Polymerase-Kettenreaktion wurde ein DNA-Abschnitt amplifiziert, welcher das opcA-Gen trägt. Hierfür wurden folgende Oligonukleotide als Primer verwendet:
opcA-A1 (SEQ ID NO: 11):
   5' cacctggcgc aggccataat 3'
opcA-E1 (SEQ ID NO: 12):
   5' cgcgtgcgcg tactacatca 3'

Die dargestellten Primer wurden von der Firma MWG Biotech (Ebersberg, Deutschland) synthetisiert. Sie ermöglichen die Amplifizierung eines ca. 1,05 kb langen DNA-Abschnittes, welcher das opcA-Gen trägt. Der Primer opcA-A1 bindet an die Region entsprechend Position 302 bis 321 des zu SEQ ID NO: 3 komplementären Stranges. Der Primer opcA-E1 bindet an die Region ensprechend position 1335 bis 1316 des Stranges gemäß SEQ ID NO: 3.

Die PCR-Reaktion wurde mit der Phusion High Fidelity DNA Polymerase (New England Biolabs, Frankfurt, Deutschland) durchgeführt. Der Reaktionsansatz wurde nach Herstellerangaben angesetzt und enthielt bei 50 µl Gesamt-Volumen 10 µl des mitgelieferten 5 x Phusion HF Buffer, Desoxynucleosidtriphosphate in einer Konzentration von jeweils 200 µM, Primer in einer Konzentration von 0,5 µM, ungefähr 50 ng Template-DNA und 2 Units Phusion Polymerase. Durch Zugabe von H₂O wurde das Volumen auf 50 µl eingestellt.

Der PCR-Ansatz wurde zuerst einer einleitenden Denaturierung bei 98°C für 30 Sekunden unterzogen. Darauf folgten sich 35x wiederholend ein Denaturierungsschritt bei 98°C für 20 Sekunden, ein Schritt zum Binden der Primer an die vorgelegte DNA bei 60°C für 20 Sekunden und der Extensionsschritt zur Verlängerung der Primer bei 72°C für 60 Sekunden. Nach dem abschliessenden Extensionsschritt für 5 Minuten bei 72°C wurde der PCR-Ansatz einer Agarosegel-Elektrophorese (0,8% Agarose) unterworfen. Ein DNA-Fragment von ca. 1,85 kb Länge wurde identifiziert, aus dem Gel isoliert und unter Verwendung des QIAquick Gel Extraction Kit der Firma Qiagen, (Hilden, Deutschland) aufgereinigt.

Die Nukleotidsequenz des amplifizierten DNA-Fragmentes bzw. PCR-Produktes wurde von der Firma Agowa (Berlin, Deutschland) bestimmt. Die erhaltene Sequenz der Kodierregion des opcA-Allels ist in der SEQ ID NO: 7 dargestellt. Die sich mit Hilfe des Programmes Patentin ergebende Aminosäuresequenz des dazugehörigen Glucose-6-Phosphat-Dehydrogenase Proteins ist in der SEQ ID NO: 8 dargestellt.

Die Nukleotidsequenz der Kodierregion des opcA-Allels der Mutante DM1825 enthält an Position 319 Cytosin, an Position 657 Cytosin, an Position 697 Thymin und an Position 781 Cytosin (Siehe SEQ ID NO: 5). Das Gen des Wildtyps (Siehe SEQ ID NO: 1) enthält an der Position 319 Thymin, an Position 657 Adenin, an Position 697 Cytosin und an Position 781 Thymin. Diese Austausche der Nukleobasen führen zu Aminosäureaustauschen an den Positionen 107, 219, 233, und 261 der resultierenden Aminosäuresequenz (Vergleiche SEQ ID NO: 2 und SEQ ID NO: 6). Diese Mutationen werden im Folgenden als opcAY107H, opcAK219N, opcAP233S und opcAY261H bezeichnet. Weiterhin enthält das opcA-Allel von DM1825 noch drei weitere Nukleotidaustausche an den Positionen 402, 600 und 648 (Siehe SEQ ID NO:7), die nicht zu einem Aminosäureaustausch führen ("stille Mutationen")

### Beispiel 4

### Konstruktion des Austauschvektors pK18mobsacB_opcAaa4ex

Mit Hilfe der Polymerase-Kettenreaktion wurde ein Fragment enthaltend die Kodierregion des opcA-Allels amplifiziert., das die Mutationen opcAY107H, opcAK219N, opcAP233S und opcAY261H trägt. Als Template wurde die in Beispiel 3 gewonnene chromosomale DNA verwendet. Als Primer wurden die in Beispiel 3 eingesetzten Oligonukleotide opcA-A1 (SEQ ID NO: 11) und opcA-E1 (SEQ ID NO: 12)ausgewählt.

Die PCR-Reaktion wurde mit der Phusion High-Fidelity DNA Polymerase (New England Biolabs, Frankfurt, Deutschland) durchgeführt. Der Reaktionsansatz hatte die oben beschriebene Zusammensetzung.. Die PCR wurde mit einer Ausnahme wie oben durchgeführt: der 72°C-Extensionsschritt in der 35-fachen Wiederholung wurde jeweils nur für 30 Sekunden durchgeführt.

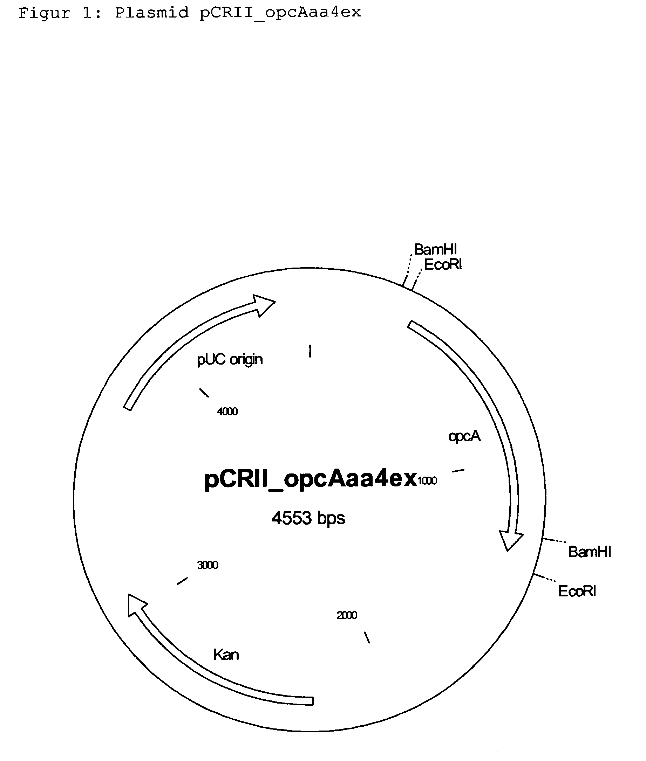

Das amplifizierte DNA Fragment von ca. 1,05 kb, welches die Kodierregion des opcA-Gens aus DM1825 trägt, wurde mit dem Zero Blunt TOPO Kit der Firma Invitrogen (Carlsbad, CA, USA) in den Vektor pCR®Blunt II-TOPO ligiert. Anschliessend wurde der E. coli Stamm Top10 (Grant et al., Proceedings of the National Academy of Sciences USA, 87 (1990) 4645-4649) mit dem Ligationsansatz nach Angaben des Kit-Herstellers (Invitrogen, Carlsbad, CA, USA) transformiert. Die Selektion von Plasmid-tragenden Zellen erfolgte durch Ausplattieren des Transformationsansatzes auf LB Agar (Merck, Darmstadt, Deutschland), der mit 25 mg/l Kanamycin supplementiert worden war. Plasmid-DNA wurde aus einer Transformante mit Hilfe des QIAprep Spin Miniprep Kit der Firma Qiagen (Hilden, Deutschland) isoliert und durch Behandlung jeweils einmal mit dem Restriktionsenzym BamHI und einmal mit dem Restriktionsenzym EcoRI mit anschliessender Agarosegel-Elektrophorese (0,8 %) überprüft. Das Plasmid wurde als pCRII_opcAaa4ex bezeichnet und ist in Figur 1 dargestellt.

Das Plasmid wurde mit der Restriktionsendonuklease EcoRI behandelt und durch Elektrophorese in einem 0,8%igen Agarosegel aufgetrennt. Das so herausgespaltene, ca. 1,05 kb große Fragment wurde anschließend aus dem Gel isoliert und mit dem QIAquick Gel Extraction Kit der Firma Qiagen aufgereinigt.

Das dergestalt gereinigte DNA-Fragment enthält die beschriebenen Mutationen in opcA und besitzt EcoRI kompatible Enden (opcAaa4ex-Fragment). Es wurde anschließend in den von Schäfer et al. (Gene, 145, 69-73 (1994) beschriebenen, mobilisierbaren Vektor pK18mobsacB eingebaut, um einen Allel- beziehungsweise Mutationsaustausch zu ermöglichen. Hierzu wurde pK18mobsacB mit dem Restriktionsenzym EcoRI verdaut und die Enden mit alkalischer Phosphatase (Alkaline Phosphatase, Boehringer Mannheim, Deutschland) dephosphoryliert. Der so vorbereitete Vektor wurde mit dem opcAaa4ex-Fragment gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al., Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2nd Ed. Cold Spring Habor, New York, 1989), der mit 25 mg/l Kanamycin supplementiert worden war.

Plasmid-DNA wurde aus einer Transformante mit Hilfe des QIAprep Spin Miniprep Kit der Firma Qiagen isoliert und durch Restriktionsspaltung jeweils einmal mit dem Enzym BamHI und einmal mit dem Enzym EcoRI und anschließender Agarosegel-Elektrophorese überprüft. Das Plasmid wurde pK18mobsacB_opcAaa4ex genannt und ist in Figur 2 dargestellt.

### Beispiel 5

### Einbau der Mutationen opcAY107H, opcAK219N, opcAP233S und opcAY261H in den Stamm DM1797

Der in Beispiel 4 beschriebene Vektor pK18mobsacB_ opcAaa4ex wurde nach dem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_opcAaa4ex erfolgte durch Ausplattieren des Konjugationsansatzes auf LB-Agar; der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_opcAaa4ex enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, die die Synthese des für C. glutamicum toxischen Produktes Levan katalysiert. Auf mit Sucrose supplementiertem LB-Agar wachsen daher nur solche Klone, bei denen das integrierte pK18mobsacB_opcAaa4ex als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde ein Klon gesucht, bei dem der gewünschte Austausch, d. h. der Einbau der Mutationen opcAY107H, opcAK219N, opcAP233S und opcAY261H, erfolgt war. Hierzu wurde von 20 Klonen mit dem Phänotyp "Wachstum in Gegenwart von Sucrose" und "Nicht-Wachstum in Gegenwart von Kanamycin" die Sequenz des opcA-Gens bestimmt.. Auf diese Weise wurde ein Klon identifiziert, der die Mutationen opcAY107H, opcAK219N, opcAP233S und opcAY261H trägt. Dieser Stamm wurde als C. glutamicum DM1797_opcAaa4ex bezeichnet.

### Beispiel 6

### Vergleich der Leistung des Stammes DM1797_opcAaa4ex mit der des Ausgangsstammes DM1797

Der Leistungtest wurde wie in Beispiel 2 beschrieben durchgeführt. Der Stamm DM1797_opcAaa4ex zeigte im Vergleich zu DM1797 eine erhöhte Lysinausscheidung ähnlich wie DM1825 (Siehe Tabelle 1).

Kurze Beschreibung der Figuren:
- Figur 1:: Karte des Plasmids pCRII_opcAaa4ex
- Figur 2:: Karte des Plasmids pK18mobsacB_opcAaa4ex

Die verwendeten Abkürzungen und Bezeichnungen haben folgende Bedeutung. Bei der Angabe der Basenpaarzahl (bps) handelt es sich um Näherungswerte, die im Rahmen der Reproduzierbarkeit von Messungen erhalten werden..
- pUC: Replikationsursprung pUC
- Kan:: Kanamycin Resistenz-Gen
- EcoRI:: Schnittstelle des Restriktionsenzyms EcoRI
- BamHI:: Schnittstelle des Restriktionsenzyms BamHI
- opcA:: kloniertes DNA-Fragment enthaltend die Kodierregion des opcA-Allels der Mutante DM1825
- sacB:: sacB-Gen
- RP4-mob:: mob-Region mit dem Replikationsursprung für den Transfer (oriT)
- oriV:: Replikationsursprung V

## Patentansprüche

1. Isolierte Mutanten coryneformer Bakterien, welche ein Gen enthalten, das für das Polypeptid Glucose-6-Phosphat-Dehydrogenase kodiert, wobei die Aminosäuresequenz des Polypeptids, ausgewählt ist aus der Gruppe, bestehend aus
a) SEQ ID NO: 6, in der sich
an Position 107 der Aminosäuresequenz L-Histidin
an Position 219 L-Asparagin,
an Position 233 L-Serin und
an Position 261 L-Histidin befinden,
b) SEQ ID NO: 6, wobei sich über die genannten Mutationen hinaus maximal 5 konservative Aminosäureaustausche finden, und
c) SEQ ID NO: 6, wobei sich über genannten Mutationen hinaus maximal 5 Insertionen oder Deletionen finden.

2. Mutanten coryneformer Bakterien gemäß Anspruch 1, **dadurch gekennzeichnet, dass** es sich bei den coryneformen Bakterien um ein Bakterium ausgewählt aus der Gruppe Corynebacterium efficiens, Corynebacterium glutamicum, Corynebacterium thermoaminogenes und Corynebacterium aminogenes handelt.

3. Mutanten coryneformer Bakterien gemäß Anspruch 2, **dadurch gekennzeichnet, dass** es sich um Corynebacterium glutamicum handelt.

4. Mutanten coryneformer Bakterien gemäß den Ansprüchen 1, 2, oder 3, **dadurch gekennzeichnet, dass** es sich um L-Aminosäure ausscheidende Bakterien handelt.

5. Mutanten coryneformer Bakterien gemäß Anspruch 4, **dadurch gekennzeichnet, dass** es sich um L-Lysin oder um L-Tryptophan ausscheidende Bakterien handelt.

6. Mutanten coryneformer Bakterien gemäß einem oder mehreren der Ansprüche 1 bis 5, **dadurch gekennzeichnet, dass** das Gen eine Nukleotidsequenz umfasst, die mit der Nukleotidsequenz eines Polynukleotids identisch ist, das durch eine Polymerasekettenreaktion (PCR) unter Verwendung von DNA gewonnen aus einem coryneformen Bakterium und unter Verwendung eines Primerpaares bestehend aus einem ersten Primer, umfassend mindestens 15 aufeinanderfolgende Nukleotide ausgewählt aus der Nukleotidsequenz zwischen Position 1 und 334 von SEQ ID NO:3, und einem zweitem Primer, umfassend mindestens 15 aufeinanderfolgende Nukleotide ausgewählt aus der komplementären Nukleotidsequenz zwischen Position 1600 und 1292 von SEQ ID NO:3, erhältlich ist.

7. Mutanten coryneformer Bakterien gemäß einem oder mehreren der Ansprüche 1 bis 5, **dadurch gekennzeichnet, dass** das Gen eine Nukleotidsequenz umfasst, die mindestens 90% identisch ist mit den Nukleotidsequenzen von SEQ ID NO:5 oder SEQ ID NO:7.

8. Mutanten coryneformer Bakterien gemäß den Ansprüchen 6 und 7, **dadurch gekennzeichnet, dass** das Gen die Nukleotidsequenz, die zu 100 % identisch ist mit der von SEQ ID NO:5 oder SEQ ID NO:7, umfasst.

9. Isoliertes Polynukleotid, das für das Polypeptid der Glucose-6-Phosphat-Dehydrogenase kodiert, wobei die Aminosäuresequenz des Polypeptids, ausgewählt ist aus der Gruppe, bestehend aus
a) SEQ ID NO: 6, in der sich an Position 107 der Aminosäuresequenz L-Histidin
an Position 219 L-Asparagin
an Position 233 L-Serin und
an Position 261 L-Histidin befinden,
b) SEQ ID NO: 6, und sich über die genannten Mutationen hinaus maximal 5 konservative Aminosäureaustausche, und
c) SEQ ID NO: 6, in der sich über genannten Mutationen hinaus maximal 5 Insertionen oder Deletionen finden.

10. Isoliertes Polynukleotid gemäß Anspruch 9, **dadurch gekennzeichnet, dass** es mit der Nukleotidsequenz eines Polynukleotids identisch ist, das durch eine Polymerasekettenreaktion (PCR) unter Verwendung von DNA gewonnen aus einem coryneformen Bakterium und unter Verwendung eines Primerpaares bestehend aus einem ersten Primer, umfassend mindestens 15 aufeinanderfolgende Nukleotide ausgewählt aus der Nukleotidsequenz zwischen Position 1 und 334 von SEQ ID NO:3, und einem zweitem Primer, umfassend mindestens 15 aufeinanderfolgende Nukleotide ausgewählt aus der komplementären Nukleotidsequenz zwischen Position 1600 und 1292 von SEQ ID NO:3, erhältlich ist.

11. Isoliertes Polynukleotid gemäß Anspruch 9, **dadurch gekennzeichnet, dass** es mit eines zu SEQ ID NO:5 komplementären Nukleotidsequenz unter stringenten Bedingungen hybridisiert, wobei die stringenten Bedingungen einen Waschschritt mit einem Puffer entsprechend 2xSSC bei einer Temperatur von 52°C bis 68°C umfassen.

12. Isoliertes Polynukleotid gemäß Anspruch 9, **dadurch gekennzeichnet, dass** das Polynukleotid eine Nukleotidsequenz umfasst, die mindestens 90% identisch ist mit der Nukleotidsequenz von SEQ ID NO:5 oder SEQ ID NO:7.

13. Isoliertes Polynukleotid gemäß Anspruch 9, **dadurch gekennzeichnet, dass** das Polynukleotid eine Nukleotidsequenz umfasst, die mindestens 96% identisch ist mit der Nukleotidsequenz von SEQ ID NO: 5.

14. Isoliertes Polynukleotid gemäß Anspruch 12, **dadurch gekennzeichnet, dass** die Nukleotidsequenzen SEQ ID NO:5 oder SEQ ID NO:7 umfassen.

15. Isoliertes Polynukleotid, **dadurch gekennzeichnet, dass** es mindestens für ein Leseraster mit einer Aminosäuresequenz entsprechend den Positionen 98 bis 270 von SEQ ID NO:6 kodiert.

16. Isoliertes Polynukleotid gemäß Anspruch 15, **dadurch gekennzeichnet, dass** es eine oder mehrere stille Mutationen enthält.

17. Isoliertes Polynukleotid gemäß den Ansprüchen 15 oder 16, **dadurch gekennzeichnet, dass** es mindestens die Nukleotidsequenz entsprechend den Positionen 292 bis 810 von SEQ ID NO:5 oder SEQ ID NO:7 umfasst.

18. Ein rekombinanter Mikroorganismus, der das isolierte Polynukleotid gemäß den Ansprüchen 9 bis 17 enthält, oder unter dessen Verwendung hergestellt wurde.

19. Rekombinanter Mikroorganismus gemäß Anspruch 18, **dadurch gekennzeichnet, dass** es sich um ein coryneformes Bakterium oder um ein Bakterium der Gattung Escherichia handelt.

20. Rekombinanter Mikroorganismus gemäß Anspruch 19, **dadurch gekennzeichnet, dass** es sich bei dem coryneformen Bakterium um die Gattung Corynebacterium handelt.

21. Rekombinanter Mikroorganismus gemäß Anspruch 20, **dadurch gekennzeichnet, dass** es sich bei dem Bakterium der Gattung Corynebacterium um die Art Corynebacterium glutamicum handelt.

22. Ein Vektor, der das isolierte Polynukleotid gemäß den Ansprüchen 9 bis 17 enthält.

23. Ein rekombinanter Mikroorganismus, der den Vektor gemäß Anspruch 22 enthält.

24. Rekombinanter Mikroorganismus gemäß Anspruch 23, **dadurch gekennzeichnet, dass** es sich um ein coryneformes Bakterium oder um ein Bakterium der Gattung Escherichia handelt.

25. Rekombinanter Mikroorganismus gemäß Anspruch 24, **dadurch gekennzeichnet, dass** es sich bei dem coryneformen Bakterium um die Gattung Corynebacterium handelt.

26. Rekombinanter Mikroorganismus gemäß Anspruch 25, **dadurch gekennzeichnet, dass** es sich bei dem Bakterium der Gattung Corynebacterium um die Art Corynebacterium glutamicum handelt.

27. Rekombinantes coryneformes Bakterium, welches in seinem Chromosom ein Polynukleotid gemäß Anspruch 9 enthält, wobei das genannte Polynukleotid durch Verknüpfung mit einem Promoter überexprimiert wird

28. Verfahren zur Herstellung einer L-Aminosäure, **dadurch gekennzeichnet, dass** man ein isoliertes coryneformes Bakterium gemäß den Ansprüchen 1-8, 18-21, 23-27 in einem geeigneten Medium fermentiert.

29. Verfahren gemäß Anspruch 28, **dadurch gekennzeichnet, dass** man die gewünschte L-Aminosäure isoliert oder sammelt

30. Verfahren gemäß Anspruch 29, **dadurch gekennzeichnet, dass** man die gewünschte L-Aminosäure reinigt.

31. Verfahren gemäß den Ansprüchen 28 bis 30, **dadurch gekennzeichnet, dass** man die gewünschte L-Aminosäure gemeinsam mit Bestandteilen aus der Fermentationsbrühe und/oder der Biomasse (> 0 bis 100 %) isoliert oder sammelt.

32. Verfahren gemäß Anspruch 28, **dadurch gekennzeichnet, dass** man
a) aus der Fermentationsbrühe die gebildete Biomasse in einer Menge von 0 bis 100 % entfernt, und
b) aus der so erhaltenen Brühe ein im wesentlichen trockenes und geformtes Produkt durch eine Methode ausgewählt aus der Gruppe Granulation, Kompaktierung, Sprühtrocknung und Extrusion herstellt.

33. Verfahren nach Anspruch 32, **dadurch gekennzeichnet, dass** man der Fermentationsbrühe vor oder nach Schritt a) eine Säure ausgewählt aus der Gruppe Schwefelsäure, Salzsäure und Phosphorsäure hinzufügt.

34. Verfahren nach Anspruch 32, **dadurch gekennzeichnet, dass** man aus der vor oder nach in Schritt a) erhaltenen Brühe Wasser entfernt.

35. Verfahren gemäß Schritt 32, **dadurch gekennzeichnet, dass** man das in oder während Schritt b) erhaltene geformte Produkt mit einem Öl besprüht.

36. Stamm DM 1825 von Corynebacterium glutamicum, hinterlegt unter der Nr. DSM 17223 bei der DSMZ (Braunschweig).

## Claims

1. Isolated mutants of coryneform bacteria, which comprise a gene coding for the polypeptide of glucose 6-phosphate dehydrogenase, where the amino acid sequence of said polypeptide is selected from the group consisting of
a) SEQ ID NO:6 which contains
L-histidine in position 107 of the amino acid sequence
L-asparagine in position 219
L-serine in position 233, and
L-histidine in position 261,
b) SEQ ID NO: 6, wherein no more than 5 conservative amino acid substitutions are found in addition to the mutations mentioned, and
c) SEQ ID NO: 6, wherein no more than 5 insertions or deletions are found in addition to the mutations mentioned.

2. Mutants of coryneform bacteria according to Claim 1, **characterized in that** the coryneform bacteria are a bacterium selected from the group consisting of Corynebacterium efficiens, Corynebacterium glutamicum, Corynebacterium thermoaminogenes and Corynebacterium aminogenes.

3. Mutants of coryneform bacteria according to Claim 2, **characterized in that** they are Corynebacterium glutamicum.

4. Mutants of coryneform bacteria according to Claim 1, 2 or 3, **characterized in that** they are L-amino acid-secreting bacteria.

5. Mutants of coryneform bacteria according to Claim 4, **characterized in that** they are L-lysine or L-tryptophan-secreting bacteria.

6. Mutants of coryneform bacteria according to one or more of Claims 1 to 5, **characterized in that** the gene comprises a nucleotide sequence which is identical to the nucleotide sequence of a polynucleotide obtainable by a polymerase chain reaction (PCR) using DNA obtained from a coryneform bacterium and using a primer pair consisting of a first primer comprising at least 15 contiguous nucleotides selected from the nucleotide sequence between positions 1 and 334 of SEQ ID NO: 3, and of a second primer comprising at least 15 contiguous nucleotides selected from the complementary nucleotide sequence between positions 1600 and 1292 of SEQ ID NO: 3.

7. Mutants of coryneform bacteria according to one or more of Claims 1 to 5, **characterized in that** the gene comprises a nucleotide sequence which is at least 90% identical to the nucleotide sequences of SEQ ID NO: 5 or SEQ ID NO: 7.

8. Mutants of coryneform bacteria according to Claims 6 and 7, **characterized in that** the gene comprises the nucleotide sequence which is 100% identical to that of SEQ ID NO: 5 or SEQ ID NO: 7.

9. Isolated polynucleotide encoding the polypeptide of glucose 6-phosphate dehydrogenase, where the amino acid sequence of said polypeptide is selected from the group consisting of
a) SEQ ID NO:6 which contains L-histidine in position 107 of the amino acid sequence
L-asparagine in position 219 L-serine in position 233, and
L-histidine in position 261,
b) SEQ ID NO: 6, wherein no more than 5 conservative amino acid substitutions are found in addition to the mutations mentioned, and
c) SEQ ID NO: 6, wherein no more than 5 insertions or deletions are found in addition to the mutations mentioned.

10. Isolated polynucleotide according to Claim 9, **characterized in that** it is identical to the nucleotide sequence of a polynucleotide obtainable by a polymerase chain reaction (PCR) using DNA obtained from a coryneform bacterium and using a primer pair consisting of a first primer comprising at least 15 contiguous nucleotides selected from the nucleotide sequence between positions 1 and 334 of SEQ ID NO: 3, and of a second primer comprising at least 15 contiguous nucleotides selected from the complementary nucleotide sequence between positions 1600 and 1292 of SEQ ID NO: 3.

11. Isolated polynucleotide according to Claim 9, **characterized in that** is hybridizes with a nucleotide sequence complementary to SEQ ID NO: 5 under stringent conditions, said stringent conditions comprising a washing step using a buffer corresponding to 2 × SSC at a temperature of from 52°C to 68°C.

12. Isolated polynucleotide according to Claim 9, **characterized in that** the polynucleotide comprises a nucleotide sequence which is at least 90% identical to the nucleotide sequence of SEQ ID NO: 5 or SEQ ID NO: 7.

13. Isolated polynucleotide according to Claim 9, **characterized in that** the polynucleotide comprises a nucleotide sequence which is at least 96% identical to the nucleotide sequence of SEQ ID NO: 5.

14. Isolated polynucleotide according to Claim 12, **characterized in that** the nucleotide sequences comprise SEQ ID NO: 5 or SEQ ID NO: 7.

15. Isolated polynucleotide, **characterized in that** it encodes at least one reading frame having an amino acid sequence corresponding to positions 98 to 270 of SEQ ID NO: 6.

16. Isolated polynucleotide according to Claim 15, **characterized in that** it comprises one or more silent mutations.

17. Isolated polynucleotide according to Claim 15 or 16, **characterized in that** it comprises at least the nucleotide sequence corresponding to positions 292 to 810 of SEQ ID NO: 5 or SEQ ID NO: 7.

18. A recombinant microorganism comprising the isolated polynucleotide according to Claims 9 to 17 or prepared using the same.

19. Recombinant microorganism according to Claim 18, **characterized in that** it is a coryneform bacterium or a bacterium of the genus Escherichia.

20. Recombinant microorganism according to Claim 19, **characterized in that** the coryneform bacterium is the genus Corynebacterium.

21. Recombinant microorganism according to Claim 20, **characterized in that** the bacterium of the genus Corynebacterium is the species Corynebacterium glutamicum.

22. A vector comprising the isolated polynucleotide according to Claims 9 to 17.

23. A recombinant microorganism comprising the vector according to Claim 22.

24. Recombinant microorganism according to Claim 23, **characterized in that** it is a coryneform bacterium or a bacterium of the genus Escherichia.

25. Recombinant microorganism according to Claim 24, **characterized in that** the coryneform bacterium is the genus Corynebacterium.

26. Recombinant microorganism according to Claim 25, **characterized in that** the bacterium of the genus Corynebacterium is the species Corynebacterium glutamicum.

27. Recombinant coryneform bacterium comprising in its chromosome a polynucleotide according to Claim 9, wherein said polynucleotide is overexpressed by being linked to a promoter.

28. Process for preparing an L-amino acid, **characterized in that** an isolated coryneform bacterium according to Claims 1-8, 18-21, 23-27 is fermented in a suitable medium.

29. Process according to Claim 28, **characterized in that** the desired L-amino acid is isolated or collected.

30. Process according to Claim 29, **characterized in that** the desired L-amino acid is purified.

31. Process according to Claims 28 to 30, **characterized in that** the desired L-amino acid is isolated or collected together with components of the fermentation broth and/or the biomass (> 0 to 100%).

32. Process according to Claim 28, **characterized in that**
a) an amount of from 0 to 100% of the biomass produced is removed from the fermentation broth, and
b) from the broth obtained in this way an essentially dry and shaped product is prepared by a method selected from the group consisting of granulation, compacting, spray drying and extrusion.

33. Process according to Claim 32, **characterized in that** an acid selected from the group consisting of sulfuric acid, hydrochloric acid and phosphoric acid is added to the fermentation broth prior to or after step a).

34. Process according to Claim 32, **characterized in that** water is removed from the broth obtained prior to or after in step a).

35. Process according to Claim 32, **characterized in that** the shaped product obtained in or during step b) is sprayed with an oil.

36. Corynebacterium glutamicum strain DM 1825, deposited with the DSMZ (Brunswick, Germany) under No. DSM 17223.

## Revendications

1. Mutants isolés de bactéries corynéformes, qui contiennent un gène qui code pour le polypeptide de la glucose-6-phosphate-déshydrogénase, la séquence d'acides aminés du polypeptide étant choisie dans le groupe consistant en :
a) SEQ ID N° 6, dans laquelle on a
une L-histidine sur la position 107 de la séquence d'acides aminés,
une L-asparagine sur la position 219,
une L-sérine sur la position 233, et
une L-histidine sur la position 261,
b) SEQ ID N° 6, dans laquelle au plus 5 échanges d'acides aminés conservateurs existent au-delà des mutations mentionnées,
c) SEQ ID N° 6, dans laquelle au plus 5 insertions ou délétions existent au-delà des mutations mentionnées.

2. Mutants de bactéries corynéformes selon la revendication 1, **caractérisés en ce que**, pour ce qui concerne les bactéries corynéformes, il s'agit d'une bactérie choisie dans le groupe consistant en Corynebacterium efficiens, Corynebacterium glutamicum, Corynebacterium thermoaminogenes et Corynebacterium aminogenes.

3. Mutants de bactéries corynéformes selon la revendication 2, **caractérisés en ce qu'**il s'agit de Corynebacterium glutamicum.

4. Mutants de bactéries corynéformes selon les revendications 1, 2 ou 3, **caractérisés en ce qu'**il s'agit de bactéries produisant un acide L-aminé.

5. Mutants de bactéries corynéformes selon la revendication 4, **caractérisés en ce qu'**il s'agit de bactéries produisant de la L-lysine ou du L-tryptophane.

6. Mutants de bactéries corynéformes selon l'une ou plusieurs des revendications 1 à 5, **caractérisés en ce que** le gène comprend une séquence nucléotidique qui est identique à la séquence nucléotidique d'un polynucléotide qui peut être obtenu par une réaction en chaîne par polymérase (PCR) par utilisation d'un ADN obtenu à partir d'une bactérie corynéforme et par utilisation d'une paire d'amorces constituée d'une première amorce comprenant au moins 15 nucléotides successifs choisis dans la séquence nucléotidique entre la position 1 et la position 334 de SEQ ID N° 3, et d'une deuxième amorce, comprenant au moins 15 nucléotides successifs choisis dans la séquence nucléotidique complémentaire entre la position 1600 et la position 1292 de SEQ ID N° 3.

7. Mutants de bactéries corynéformes selon l'une ou plusieurs des revendications 1 à 5, **caractérisés en ce que** le gène comprend une séquence nucléotidique qui a une identité d'au moins 90 % avec les séquences nucléotidiques de SEQ ID N°5 ou SEQ ID N° 7.

8. Mutants de bactéries corynéformes selon les revendications 6 et 7, **caractérisés en ce que** le gène comprend la séquence nucléotidique qui a une identité de 100 % avec celle de SEQ ID N° 5 ou SEQ ID N° 7.

9. Polynucléotide isolé, qui code pour le polypeptide de la glucose-6-phosphate-déshydrogénase, la séquence d'acides aminés du polypeptide étant choisie dans le groupe consistant en :
a) SEQ ID N° 6, dans laquelle on a
une L-histidine sur la position 107 de la séquence d'acides aminés,
une L-asparagine sur la position 219,
une L-sérine sur la position 233, et
une L-histidine sur la position 261,
b) SEQ ID N° 6, dans laquelle au plus 5 échanges d'acides aminés conservateurs existent au-delà des mutations mentionnées,
c) SEQ ID N° 6, dans laquelle au plus 5 insertions ou délétions existent au-delà des mutations mentionnées.

10. Polynucléotide isolé selon la revendication 9, **caractérisé en ce qu'**il est identique à la séquence nucléotidique d'un polynucléotide qui peut être obtenu par une réaction en chaîne par polymérase (PCR) par utilisation d'un ADN obtenu à partir d'une bactérie corynéforme et par utilisation d'une paire d'amorces constituée d'une première amorce comprenant au moins 15 nucléotides successifs choisis dans la séquence nucléotidique entre la position 1 et la position 334 de SEQ ID N° 3 et d'une deuxième amorce comprenant au moins 15 nucléotides successifs choisis dans la séquence nucléotidique complémentaire entre la position 1600 et la position 1292 de SEQ ID N° 3.

11. Polynucléotide isolé selon la revendication 9, **caractérisé en ce qu'**il s'hybride dans des conditions stringentes à une séquence nucléotidique complémentaire de SEQ ID N° 5, les conditions stringentes comprenant une étape de lavage avec un tampon correspondant à 2xSSC à une température de 52 à 68°C.

12. Polynucléotide isolé selon la revendication 9, **caractérisé en ce que** le polynucléotide comprend une séquence nucléotidique qui a une identité d'au moins 90 % avec la séquence nucléotidique de SEQ ID N° 5 ou SEQ ID N° 7.

13. Polynucléotide isolé selon la revendication 9, **caractérisé en ce que** le polynucléotide comprend une séquence nucléotidique qui a une identité d'au moins 96 % avec la séquence nucléotidique de SEQ ID N° 5.

14. Polynucléotide isolé selon la revendication 12, **caractérisé en ce que** les séquences nucléotidiques comprennent SEQ ID N° 5 ou SEQ ID N°7.

15. Polynucléotide isolé, **caractérisé en ce qu'**il code pour au moins un cadre de lecture ayant une séquence d'acides aminés correspondant aux positions 98 à 270 de SEQ ID N° 6.

16. Polynucléotide isolé selon la revendication 15, **caractérisé en ce qu'**il contient une ou plusieurs mutations silencieuses.

17. Polynucléotide isolé selon les revendications 15 ou 16, **caractérisé en ce qu'**il comprend au moins la séquence nucléotidique correspondant aux positions 292 à 810 de SEQ ID N°5 ou de SEQ ID N°7.

18. Micro-organisme recombinant, qui contient le polynucléotide isolé selon les revendications 9 à 17, ou qui a été préparé par utilisation de ce dernier.

19. Micro-organisme recombinant selon la revendication 18, **caractérisé en ce qu'**il s'agit d'une bactérie corynéforme ou d'une bactérie du genre Escherichia.

20. Micro-organisme recombinant selon la revendication 19, **caractérisé en ce que**, pour ce qui concerne la bactérie corynéforme, il s'agit du genre Corynebacterium.

21. Micro-organisme recombinant selon la revendication 20, **caractérisé en ce que**, pour ce qui concerne la bactérie du genre Corynebacterium, il s'agit de l'espèce Corynebacterium glutamicum.

22. Vecteur qui contient le polynucléotide isolé selon les revendications 9 à 17.

23. Micro-organisme recombinant qui contient le vecteur selon la revendication 22.

24. Micro-organisme recombinant selon la revendication 23, **caractérisé en ce qu'**il s'agit d'une bactérie corynéforme ou d'une bactérie du genre Escherichia.

25. Micro-organisme recombinant selon la revendication 24, **caractérisé en ce que**, pour ce qui concerne la bactérie corynéforme, il s'agit du genre Corynebacterium.

26. Micro-organisme recombinant selon la revendication 25, **caractérisé en ce que**, pour ce qui concerne la bactérie du genre Corynebacterium, il s'agit de l'espèce Corynebacterium glutamicum.

27. Bactérie corynéforme recombinante, qui dans son chromosome contient un polynucléotide selon la revendication 9, ledit polynucléotide étant surexprimé par liaison avec un promoteur.

28. Procédé de préparation d'un acide L-aminé, **caractérisé en ce qu'**on fermente dans un milieu approprié une bactérie corynéforme isolée selon les revendications 1-8, 18-21, 23-27.

29. Procédé selon la revendication 28, **caractérisé en ce qu'**on isole ou collecte l'acide L-aminé souhaité.

30. Procédé selon la revendication 29, **caractérisé en ce qu'**on purifie l'acide L-aminé souhaité.

31. Procédé selon les revendications 28 à 30, **caractérisé en ce qu'**on isole ou collecte l'acide L-aminé souhaité en même temps que des constituants du bouillon de fermentation et/ou de la biomasse (> 0 à 100 %).

32. Procédé selon la revendication 28, **caractérisé en ce que**
a) on élimine du bouillon de fermentation la biomasse formée, en une quantité de 0 à 100 %, et
b) à partir du bouillon ainsi obtenu, on prépare un produit pour l'essentiel sec et façonné, par un procédé choisi dans le groupe consistant en une granulation, un compactage, un séchage par atomisation et une extrusion.

33. Procédé selon la revendication 32 **caractérisé en ce qu'**on ajoute au bouillon de fermentation, avant ou après l'étape a), un acide choisi dans le groupe consistant en l'acide sulfurique, l'acide chlorhydrique et l'acide phosphorique.

34. Procédé selon la revendication 32, **caractérisé en ce qu'**on élimine l'eau du bouillon obtenu avant ou après l'étape a).

35. Procédé selon la revendication 32, **caractérisé en ce qu'**on pulvérise de l'huile sur le produit façonné obtenu dans l'étape b) ou pendant cette dernière.

36. Souche DM 1825 de Corynebacterium glutamicum, déposée sous le numéro DSM 17223 auprès du DSMZ (Braunschweig).
